# EUROPEAN PATENT SPECIFICATION

(11) **EP 3 568 242 B1**
(45) Date of publication and mention of the grant of the patent: **03.11.2021**
(21) Application number: 18739503.3
(22) Date of filing: 05.07.2018
(51) Int. Cl.: B07C 5/00, B29B 17/00, B09B 3/00, B03B 9/00, B03B 9/06, C10L 5/46, C12N 1/16, C12P 7/08, B29B 17/02

(54) **PROCESS AND SYSTEM FOR TREATING MUNICIPAL SOLID WASTE MATERIALS AND PRODUCING MULTIPLE PRODUCTS**
VERFAHREN UND SYSTEM ZUR BEHANDLUNG VON FESTSTOFF-SIEDLUNGSABFÄLLEN UND ZUR HERSTELLUNG VON MEHREREN PRODUKTEN
PROCÉDÉ ET SYSTÈME DE TRAITEMENT DE DÉCHETS SOLIDES MUNICIPAUX ET DE PRODUCTION DE PRODUITS MULTIPLES

(30) Priority: 05.07.2017 CA 2972505
(43) Date of publication of application: 20.11.2019
(73) Proprietor: Renasci NV, 9032 Wondelgem (BE)
(72) Inventor: DESENDER, Luc, 9032 Wondelgem (BE)
(74) Representative: De Clercq & Partners
(86) International application number: PCT/EP2018/068245
(87) International publication number: WO 2019/008094

(56) References cited:
- WO-A1-2015/090477
- WO-A2-01/89730
- WO-A2-2009/095693
- WO-A2-2014/041373
- US-A1- 2011 008 865

## Description

### TECHNICAL FIELD

The technical field generally relates to the handling and treatment of municipal solid waste (MSW), and more particularly to the conversion of such waste into products and extract recyclable materials.

### BACKGROUND

The handling of municipal solid waste (MSW) has various technical challenges. When municipal solid waste is subjected to disposal, several components that make up the waste material are lost to the disposal process.

However, MSW can be a rich source of materials and energy, which means that simple disposal or limited recycling does not adequately leverage its potential.

WO 2009/095693 A, WO 01/89730 A, US 2011/008865 A disclose the separation of MSW in different fractions.

### SUMMARY

Processes and systems disclosed herein facilitate efficient processing of MSW in order to produce products of value with efficient use and recycling of fluids and energy within the system and also to extract recyclable materials.

The processes and systems described herein facilitate operations that are energy neutral in that all of the process energy for the unit operations is derived from the MSW fed into the process. The process can be operated in energy neutral mode during normal operation after start-up operations where external energy can be used, for example.

The invention relates to a method in accordance with claim 1, and a system in accordance with claim 5.

Dependent claims refer to preferred embodiments of the invention.

In some implementations, there is provided a process for treating municipal solid waste (MSW), comprising:
separating the MSW to produce a plastics fraction, an organics fraction, a directly recyclable materials fraction and a residual fraction;
subjecting at least a portion of the plastics fraction to plastics processing to produce at least one hydrocarbon product (e.g., diesel product, paraffin product and/or hydrocarbon product suitable for use as an end product or base product in the petrochemical industry);
subjecting a first portion of the organics fraction to bio-ethanol production, comprising:
   enzymatically treating the first portion of the organics fraction to produce a sugar-enriched stream;
   fermenting at least a portion of the sugar-enriched stream to produce an ethanol containing stream; and
   separating ethanol from the ethanol containing stream to produce a bio-ethanol product;
subjecting a second portion of the organics fraction to bio-coal production, comprising:
   subjecting the second portion of the organics fraction to hydrothermal carbonization to produce a thermally treated stream (e.g., coal sludge); and
   removing water from the thermally treated stream to produce a bio-coal product;
separating the directly recyclable materials fraction into a ferro-materials fraction and a non ferro-materials fraction, and recycling the same; and
supplying at least a portion of the residuals fraction to a thermal reactor (e.g., fluid sand bed reactor or another type of similar reactor that can include a fluidized bed) to produce an inert material.

In some implementations, the plastics processing comprises melting, cracking, quenching and fractionally distilling to produce the diesel product as well as additional hydrocarbon cuts.

In some implementations, the process includes subjecting part of the sugar-enriched stream to isobutanol production to produce an isobutanol stream, and reacting at least a portion of the isobutanol stream with glycerol to produce Glycerol Tert-Butyl Ethers (GTBE).

In some implementations, the removing of water from the thermally treated stream comprises dewatering followed by drying to produce the bio-coal product and recovered water.

In some implementations, the recovered water is treated to produce fertilization material and treated water.

In some implementations, the treated water and/or the recovered water is reused in the bio-coal production and/or other parts of the process.

In some implementations, there is provided a process for treating municipal solid waste (MSW), comprising:
separating the MSW to produce a plastics fraction and an organics fraction;
subjecting at least a portion of the plastics fraction to plastics processing to produce at least one diesel product; and
subjecting at least a portion of the organics fraction to bio-ethanol production, comprising:
   enzymatically treating the first portion of the organics fraction to produce a sugar-enriched stream;
   fermenting at least a portion of the sugar-enriched stream to produce an ethanol containing stream; and
   separating ethanol from the ethanol containing stream to produce a bio-ethanol product.

In some implementations, the process includes subjecting a second portion of the organics fraction to bio-coal production. In some implementations, the bio-coal production comprises: subjecting the second portion of the organics fraction to hydrothermal carbonization to produce a thermally treated stream; and removing water from the thermally treated stream to produce a bio-coal product. In some implementations, the removing of water from the thermally treated stream comprises dewatering followed by drying to produce the bio-coal product and recovered water. In some implementations, the recovered water is treated to produce fertilization material and treated water. In some implementations, the treated water and/or the recovered water is reused in the bio-coal production. In some implementations, the plastics processing comprises melting at least a portion of the plastics fraction to produce a melted plastics stream. In some implementations, the plastics processing further comprises subjecting the melted plastics stream to cracking to produce a cracked hydrocarbon stream. In some implementations, the plastics processing further comprises quenching the cracked hydrocarbon stream to produce a quenched stream. In some implementations, the plastics processing further comprises distilling the quenched stream to produce the diesel product. In some implementations, the distilling comprises fractional distillation configured and operated to produce the diesel product as well as additional hydrocarbon cuts. In some implementations, a portion of the plastics fraction is supplied to a thermal reactor (e.g., scavenger unit) to produce inert material and also generate energy. In some implementations, the thermal reactor comprises a fluidized sand bed reactor. In some implementations, the separating of the MSW includes producing a residuals fraction, and wherein the thermal reactor is configured and operated to receive and convert the residuals fraction into at least part of the inert material. In some implementations, the process includes subjecting part of the sugar-enriched stream to isobutanol production to produce an isobutanol stream, and reacting at least a portion of the isobutanol stream with glycerol to produce GTBE.

In some implementations, there is provided a process for treating municipal solid waste (MSW), comprising: separating the MSW to produce a plastics fraction and an organics fraction; subjecting at least a portion of the plastics fraction to plastics processing to produce at least one hydrocarbon product (e.g., diesel); and subjecting portions of the organics fraction to bio-ethanol production and bio-coal production to produce bio-ethanol and bio-coal respectively.

In some implementations, the bio-ethanol production comprises enzymatically treating the first portion of the organics fraction to produce a sugar-enriched stream; fermenting at least a portion of the sugar-enriched stream to produce an ethanol containing stream; and separating ethanol from the ethanol containing stream to produce a bio-ethanol product. In some implementations, the bio-coal production comprises subjecting the second portion of the organics fraction to hydrothermal carbonization to produce a thermally treated stream; and removing water from the thermally treated stream to produce a bio-coal product. In some implementations, the removing of water from the thermally treated stream comprises dewatering followed by drying to produce the bio-coal product and recovered water. In some implementations, the recovered water is treated to produce fertilization material and treated water. In some implementations, the treated water or recovered water is reused in the bio-coal production. In some implementations, the plastics processing comprises melting at least a portion of the plastics fraction to produce a melted plastics stream. In some implementations, the plastics processing further comprises subjecting the melted plastics stream to cracking to produce a cracked hydrocarbon stream. In some implementations, the plastics processing further comprises quenching the cracked hydrocarbon stream to produce a quenched stream. In some implementations, the plastics processing further comprises distilling the quenched stream to produce the diesel product. In some implementations, the distilling comprises fractional distillation configured and operated to produce a diesel product and/or additional hydrocarbon cuts (e.g., naphtha cuts). In some implementations, a portion of the plastics fraction is supplied to a thermal reactor (e.g., scavenger unit) to produce inert material. In some implementations, the thermal reactor comprises a fluidized sand bed reactor. In some implementations, the separating of the MSW includes producing a residuals fraction, and wherein the thermal reactor is configured and operated to receive and convert the residuals fraction into at least part of the inert material. In some implementations, the process includes subjecting part of the sugar-enriched stream to isobutanol production to produce an isobutanol stream, and reacting at least a portion of the isobutanol stream with glycerol to produce Glycerol Tert-Butyl Ethers (GTBE).

In some implementations, the process includes recovering heat from a first process fluid and transferring at least a portion of the recovered heat to a second process fluid. In some implementations, the recovering of heat comprises feeding a hot process stream and a heat exchange fluid through a first indirect heat exchanger to produce a cooled process fluid and a heated heat exchange fluid; feeding at least a portion of the heated heat exchange fluid and a cool process fluid through a second indirect heat exchanger to produce a heated process fluid and a cooled heat exchange fluid; and recirculating at least part of the cooled heat exchange fluid as at least part of the heat exchange fluid through the first indirect heat exchanger. In some implementations, the heat exchange fluid is circulated between the first and second heat exchangers via a closed loop. In some implementations, the heat exchange fluid comprises a silicon-based fluid, a refrigerant-based fluid, or a hydrocarbon-based fluid. In some implementations, the heated heat exchange fluid is used to heat at least two distinct process fluids. In some implementations, the heating of the at least two distinct process fluids is performed sequentially or in parallel. In some implementations, the transferring of the heat is performed between the first and second process fluids in at least one indirect heat exchanger that receives both fluids.

In some implementations, there is provided a process for treating municipal solid waste (MSW), comprising:
separating the MSW to produce a plastics fraction, an organics fraction, a directly recyclable materials fraction and a residual fraction;
subjecting at least a portion of the plastics fraction to plastics-to-oil processing to produce at least one hydrocarbon product;
subjecting at least a portion of the organics fraction to bio-coal production, comprising:
   subjecting the portion of the organics fraction to hydrothermal carbonization to produce a thermally treated stream; and
   removing water from the thermally treated stream to produce a bio-coal product;
separating the directly recyclable materials fraction into a ferro-materials fraction and a non ferro-materials fraction, and recycling the same; and supplying at least a portion of the residuals fraction to a thermal reactor (e.g., fluid sand bed reactor) to produce an inert material.

In some implementations, the hydrocarbon product comprises a diesel product or another hydrocarbon cut, such as jet fuel, that can be used as an end product as a transport fuel or applied in CHP units. In some implementations, the hydrocarbon product comprises a paraffin product. In some implementations, the hydrocarbon product comprises hydrocarbon compounds suitable for use as a base product in the petrochemical industry. In some implementations, the process also includes subjecting at least a portion of the organics fraction to bio-ethanol production, comprising: enzymatically treating the first portion of the organics fraction to produce a sugar-enriched stream; fermenting at least a portion of the sugar-enriched stream to produce an ethanol containing stream; and separating ethanol from the ethanol containing stream to produce a bio-ethanol product. In some implementations, deployment of the bio-ethanol production is performed as part of a second stage of the process. In some implementations, the process includes a portion of the plastics fraction to a plastics recycling operation, comprising separating at least one plastic compound from the plastics fraction. In some implementations, the at least one plastic compound comprises PE/PP. In some implementations, the at least one plastic compound comprises PVC. In some implementations, the at least one plastic compound comprises PET. In some implementations, the at least one plastic compound comprises at least three separate categories including PET, PVC and PE/PP. In some implementations, the at least one plastic compound excludes PVC as a distinct product stream. In some implementations, the separating of the at least one plastic compound from the plastics fraction comprises near infrared (NIR) based separation in which NIR is used to identify one or more of the plastic compounds to be separated. In some implementations, the NIR based separation includes emitting NIR radiation onto the plastics fraction to produce a plastics-affected radiation (e.g., reflected), detecting the plastics-affected radiation, and removing and/or separating the plastics fraction based on the detection. In some implementations, the NIR radiation is provided at in a range of 700 to 2500 nm. In some implementations, the NIR radiation is provided at about 1656 nm and about 1724 nm. In some implementations, the NIR based separation includes a series of NIR separation stages, each stage being configured for removing a pre-determined plastic compound from the plastics fraction. In some implementations, the process includes recovering heat from the plastics-to-oil processing and reusing the recovered heat in the hydrothermal carbonization of the bio-coal production or another unit of the process. In some implementations, the heat is recovered from residue or char from the plastics-to-oil processing. In some implementations, the heat is recovered from the cracking, quenching or distillation units of the plastics-to-oil processing. In some implementations, the recovered heat is reused in the step of removing water from the thermally treated stream. In some implementations, the step of removing water from the thermally treated stream comprises dewatering followed by drying. In some implementations, the recovered heat is reused in the drying step. In some implementations, the heat is recovered from the char. In some implementations, the process includes recovering waste heat from the bio-coal production and the plastics-to-oil processing. In some implementations, the recovering of the waste heat comprises using an organic Rankine cycle system. In some implementations, the process includes recovering flue gases generated by steam generators used for heat generation (e.g., from the thermal reactor and/or gas engine), and employing at least a portion of the flue gases for progressive heat transfer to at least one steam generator and subsequently to the organic Rankine cycle system. In some implementations, the process includes recovering heat energy from the thermal reactor , and reusing the recovered heat energy in the process. In some implementations, the recovered heat energy from the thermal reactor is at least partly reused in the in the hydrothermal carbonization of the bio-coal production. In some implementations, by-product waste materials from the plastics-to-oil processing or the bio-coal production or a combination thereof, are supplied to the thermal reactor. In some implementations, the by-product waste materials comprise high caloric materials. In some implementations, a steam generator produces flue gas and steam, the steam being used for heating at least one unit in the plastics-to-oil processing, and the flue gas being used to for heating at least one unit in the bio-coal production. In some implementations, thermal oil is used as a heat exchange medium for the plastics-to-oil processing. In some implementations, a steam generator produces flue gas and steam, the steam being used for heating at least one unit in the bio-coal production, and the flue gas being used to for heating at least one unit in the plastics-to-oil processing. In some implementations, the bio-coal production includes a first steam generator that produces a first steam output, part of which is supplied to the hydrothermal carbonization step of the bio-coal production; and the plastics-to-oil processing includes a second steam generator that produces a second steam output, at least part of which is supplied to the plastics-to-oil processing. In some implementations, flue gas streams from both first and second steam generators are supplied to heat to a third steam generator that produces a third steam output that is used in the process. In some implementations, at least part of the third steam output is combined with the first steam output or supplied to the hydrothermal carbonization step of the bio-coal production. In some implementations, the flue gas streams are supplied from the third steam generator to an organic Rankine cycle system to provide residual heat thereto. In some implementations, one or more of the steam generators uses a fuel derived from the MSW.

In some implementations, there is provided a system for treating municipal solid waste (MSW), comprising:
a separation unit for separating the MSW to produce a plastics fraction, an organics fraction, a directly recyclable ferro-materials fraction, a directly recyclable non-ferro-materials and a residual fraction;
a plastics-to-oil processing unit for converting at least a portion of the plastics fraction to into at least one hydrocarbon product;
a bio-coal production unit for receiving at least a portion of the organics fraction to produce bio-coal, the bio-coal production unit comprising:
   a hydrothermal carbonization unit that receives the portion of the organics fraction to produce a thermally treated stream; and
   a water removal unit that receives the thermally treated stream to produce a bio-coal product and a recovered water stream;
recycling the directly recyclable ferro-materials fraction and the directly recyclable non-ferro-materials; and
a thermal reactor (e.g., scavenger unit) receiving the residuals fraction and producing an inert material and optionally generate energy

In some implementations, the thermal reactor can be a fluid sand bed reactor or similar type of reactor. In some implementations, the hydrocarbon product comprises a diesel product or another hydrocarbon cut, such as jet fuel, that can be used as an end product as a transport fuel or applied in CHP units. In some implementations, the hydrocarbon product comprises a paraffin product. In some implementations, the hydrocarbon product comprises hydrocarbon compounds suitable for use as a base product in the petrochemical industry. In some implementations, the system includes a bio-ethanol production unit for receiving a portion of the organics fraction to produce bio-ethanol. In some implementations, the bio-ethanol production unit comprises an enzymatic treatment unit for enzymatically treating the portion of the organics fraction to produce a sugar-enriched stream; a fermentation unit for fermenting at least a portion of the sugar-enriched stream to produce an ethanol containing stream; and an ethanol separator for separating ethanol from the ethanol containing stream to produce a bio-ethanol product. In some implementations, the bio-ethanol production unit is deployable as part of a second stage and deployment of an initial installation. In some implementations, the system includes a plastics recycle unit for subjecting a portion of the plastics fraction to a plastics recycling operation in which at least one plastic compound is separated from the plastics fraction. In some implementations, the at least one plastic compound comprises PE/PP. In some implementations, the at least one plastic compound comprises PVC. In some implementations, the at least one plastic compound comprises PET. In some implementations, the at least one plastic compound comprises at least three separate categories including PET, PVC and PE/PP. In some implementations, the plastics recycle unit comprises a near infrared (NIR) based separation device in which NIR is used to identify one or more of the plastic compounds to be separated. In some implementations, the NIR based separation device includes an NIR emission module configured for emitting NIR radiation onto the plastics fraction to produce a plastics-affected radiation (e.g., reflected), a detection module for detecting the plastics-affected radiation, and a separation module for separating the plastics fraction based on the detection. In some implementations, the NIR radiation is provided at in a range of 700 to 2500 nm. In some implementations, the NIR radiation is provided at about 1656 nm and about 1724 nm. In some implementations, the NIR based separation device includes a series of NIR separation stages, each stage being configured for separating a pre-determined plastic compound from the plastics fraction. In some implementations, the system has a heat recovery system for recovering heat from the plastics-to-oil processing unit and reusing the recovered heat in the hydrothermal carbonization unit. In some implementations, the heat recovery system is configured to recover heat from residue or ash produced by the plastics-to-oil processing unit. In some implementations, the heat recovery system is coupled to cracking, quenching and\or distillation units of the plastics-to-oil processing unit for recovering heat therefrom. In some implementations, the heat recovery system is coupled to the water removal unit to use the recovered heat therein. In some implementations, the water removal unit comprises a dewatering unit followed by drying unit. In some implementations, the heat recovery system is coupled to the drying unit to aid in drying. In some implementations, the heat recovery system is configured to recover the heat from the char produced by the plastics-to-oil processing unit. In some implementations, the system includes a waste heat recovery system for recovering waste heat from the bio-coal production and the plastics-to-oil processing. In some implementations, the waste heat recovery system comprises an organic Rankine cycle system. In some implementations, the system has a flue gas recovery system for recovering flue gases generated by steam generators used for heat generation, the flue gas recovery system being configured to employ at least a portion of the flue gases for progressive heat transfer to at least one steam generator and subsequently to the organic Rankine cycle system. In some implementations, the system has a heat energy recovery assembly for recovering heat energy from the thermal reactor, and reusing the recovered heat energy in the process. In some implementations, the heat energy recovery assembly is configured to at least partly reuse the heat energy in the in the hydrothermal carbonization unit. In some implementations, the system has a waste material transfer assembly for transferring by-product waste materials from the plastics-to-oil processing or the bio-coal production or a combination thereof, to the thermal reactor. In some implementations, the by-product waste materials comprise high caloric materials. In some implementations, the bio-coal production unit includes a first steam generator that produces a first steam output, part of which is supplied to the hydrothermal carbonization unit of the bio-coal production unit; and the plastics-to-oil processing unit includes a second steam generator that produces a second steam output, at least part of which is supplied to the plastics-to-oil processing unit. In some implementations, flue gas streams from both first and second steam generators are supplied to heat to a third steam generator that produces a third steam output that is used in the process. In some implementations, at least part of the third steam output is combined with the first steam output or supplied to the hydrothermal carbonization unit. In some implementations, the flue gas streams are supplied from the third steam generator to an organic Rankine cycle system to provide residual heat thereto. In some implementations, one or more of the steam generators uses a fuel derived from the MSW.

In some implementations, there is provided a system for treating municipal solid waste (MSW), comprising:
a separation unit for separating the MSW to produce a plastics fraction, an organics fraction, a ferro-materials fraction, a non ferro-materials fraction, and a residual fraction;
a plastics-to-oil processing unit for converting at least a portion of the plastics fraction to into at least one hydrocarbon product;
a bio-ethanol production unit for receiving a first portion of the organics fraction to produce bio-ethanol, wherein the bio-ethanol production unit comprises:
   an enzymatic treatment unit for enzymatically treating the portion of the organics fraction to produce a sugar-enriched stream;
   a fermentation unit for fermenting at least a portion of the sugar-enriched stream to produce an ethanol containing stream; and
   an ethanol separator for separating ethanol from the ethanol containing stream to produce a bio-ethanol product.
a bio-coal production unit for receiving a second portion of the organics fraction to produce bio-coal, the bio-coal production unit comprising:
   a hydrothermal carbonization unit that receives the second portion of the organics fraction to produce a thermally treated stream; and
   a water removal unit that receives the thermally treated stream to produce a bio-coal product and a recovered water stream;
a thermal reactor (e.g., scavenger unit) receiving the residuals fraction and producing an inert material.

Such a system can also have one or more features mentioned above or herein.

In some implementations, there is provided system for treating municipal solid waste (MSW), comprising:
a separation unit for separating the MSW to produce a plastics fraction, an organics fraction, a ferro-materials fraction, a non ferro-materials fraction, and a residual fraction;
a bio-ethanol production unit for receiving a first portion of the organics fraction to produce bio-ethanol, wherein the bio-ethanol production unit comprises:
   an enzymatic treatment unit for enzymatically treating the portion of the organics fraction to produce a sugar-enriched stream;
   a fermentation unit for fermenting at least a portion of the sugar-enriched stream to produce an ethanol containing stream; and
   an ethanol separator for separating ethanol from the ethanol containing stream to produce a bio-ethanol product.
a bio-coal production unit for receiving a second portion of the organics fraction to produce bio-coal, the bio-coal production unit comprising:
   a hydrothermal carbonization unit that receives the second portion of the organics fraction to produce a thermally treated stream; and
   a water removal unit that receives the thermally treated stream to produce a bio-coal product and a recovered water stream;
a thermal reactor (e.g., scavenger unit) receiving the residuals fraction and producing an inert material and generate energy.

Such a system can also have one or more features mentioned above or herein.

In some implementations, there is provided a system for treating municipal solid waste (MSW), comprising:
a separation unit for separating the MSW to produce a plastics fraction, an organics fraction, a ferro-materials fraction, a non ferro-materials fraction, and a residual fraction;
a plastics recycling unit for recovering distinct plastic components including PET and\or PE-PP from the plastics fraction, and producing an end-of-life plastics stream;
a plastics-to-oil processing unit for converting at least a portion of the end-of-life plastics stream into at least one hydrocarbon product;
a bio-ethanol production unit for receiving a first portion of the organics fraction to produce bio-ethanol;
a bio-coal production unit for receiving a second portion of the organics fraction to produce bio-coal;
   a hydrothermal carbonization unit that receives the second portion of the organics fraction to produce a thermally treated stream; and
   a water removal unit that receives the thermally treated stream to produce a bio-coal product and a recovered water stream; and
a thermal reactor (e.g., scavenger unit) receiving the residuals fraction and producing an inert material.

Such a system can also have one or more features mentioned above or herein.

In some implementations, there is provided a process for treating a solid waste containing material, comprising: separating the solid waste containing material to produce at least a plastics fraction and a residual fraction; subjecting at least a portion of the plastics fraction to plastics processing to produce at least one hydrocarbon product; and supplying at least a portion of the residual fraction to a reactor to produce an inert material.

Such a process can also include one or more features as described above or herein. For instance, in some implementations, the separating step further produces a directly materials recyclable fraction, and optionally separating the directly recyclable materials fraction into a ferro-materials fraction and a non ferro-materials fraction. In some implementations, the separating step further produces an organic fraction. In some implementations, the process includes subjecting a portion of the organics fraction to bio-ethanol production, comprising enzymatically treating the first portion of the organics fraction to produce a sugar-enriched stream; fermenting at least a portion of the sugar-enriched stream to produce an ethanol containing stream; and separating ethanol from the ethanol containing stream to produce a bio-ethanol product. In some implementations, the process includes subjecting a portion of the organics fraction to bio-coal production, comprising subjecting the second portion of the organics fraction to hydrothermal carbonization to produce a thermally treated stream (e.g., coal sludge); and removing water from the thermally treated stream to produce a bio-coal product. In some implementations, the solid waste containing material comprises municipal solid waste (MSW). It is also noted that a corresponding system can be provided, including a separator, a plastics processing unit, and a residual fraction processing unit (e.g., thermal reactor) which may have one or more features as described herein.

In some implementations, the process is energy neutral in the sense that the needed energy to power the process comes entirely from the waste itself. By applying the thermodynamic characteristics of the flue gases in the different processes as well as heat recovery to harvest the residual heat in the flue gases, e.g., using ORCs. Various configurations can be used to ensure energy neutral operation. It is also noted that the energy neutral feature relates to operating process steps, and may not necessarily include electricity used for powering small equipment, control systems, and the like which could be powered by outside sources (e.g., an electrical grid).

It is noted that the diesel or other predefined fractions of hydrocarbons can be produced by varying and controlling process parameters to obtain specific molecular weight length ranges in the end product. Possible hydrocarbon fuels include normal fuel, naphtha, jet fuel, or diesel.

In some implementations, the bio-coal is converted into activated coal. The bio-coal material can take the form of a peat-like product, from which activated carbon can me made relatively efficiently and easily. Activated coal/carbon can lead yield a significantly higher value compared to raw bio-coal. Thus, the bio-coal production unit or step can be coupled to a operation for converting some or all of the bio-coal into activated carbon using one or more techniques for doing the same.

In some implementations, the effluent from the hydrothermal carbonization can contain a notable concentration of sugars or longer carbohydrates, and at least part of this effluent stream can be supplied for enzymatic treatment in the bio-ethanol production unit. This recycling strategy can enhance recovery of water and carbon-based compounds in the effluent for conversion into ethanol.

It is also noted that some units or steps can be replaced with alternatives, such as the use of a gasification step (e.g., rotating kiln) instead of hydrothermal carbonization. It is also noted that various features described herein can be combined with other features, implementations, units, processes, individual steps, and combinations thereof.

### BRIEF DESCRIPTION OF DRAWINGS

Fig 1 is a block diagram of a process for treating municipal solid waste.
Fig 2 is a block diagram of a sub-process for treating a plastics stream.
Fig 3 is a block diagram of a sub-process for treating another plastics stream including separating and sorting.
Fig 4 is a block diagram of a sub-process for treating an organics stream to produce bio-products such as bio-ethanol and bio-coal.
Fig 5 (A and B) is a block diagram mass balance of a process for treating municipal solid waste.
Fig 6 is a block diagram of a heat exchange configuration, which can be used in the context of the processes illustrated in Figs 1 and 5 for example.
Fig 7 is another block diagram of another heat exchange configuration with heating of multiple cool process fluids, which can be used in the context of the processes illustrated in Figs 1 and 5 for example.
Fig 8 is a block diagram of a separation process for ferro and non-ferro materials.
Fig 9 is a block diagram of optional heat recovery and power generation configurations.
Fig 10 is a block diagram of an alternative heat exchange configuration.

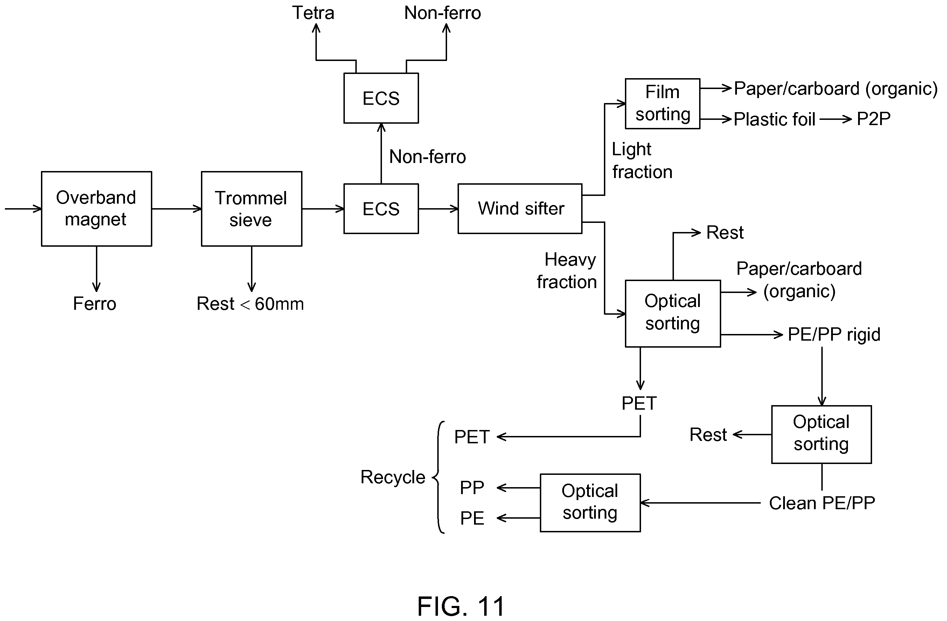

Fig 11 is a block diagram of a sorting and separation stage that is part of the overall process.
Figs 12A to 12V are block diagrams of example parts of an example process.

### DETAILED DESCRIPTION

Systems and processes are described for processing municipal solid waste (MSW) in order to recover energy and materials.

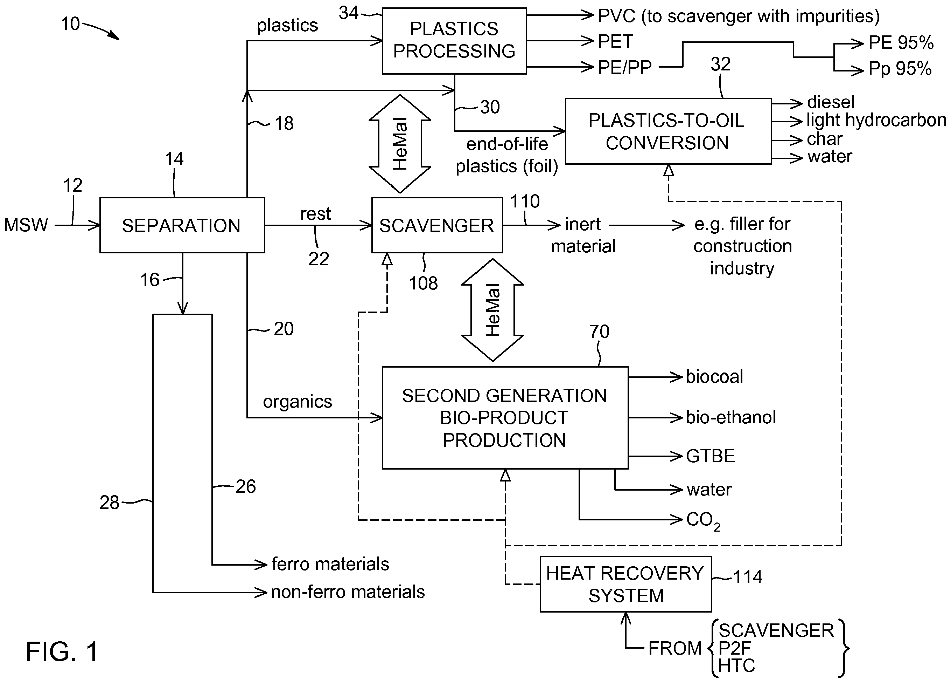

Referring to Fig 1, in one implementation, an MSW treatment process 10 includes a number of steps and for treating various components of an MSW feed stream 12 in order to produce various products.

The MSW feed stream 12 can be obtained from various sources and includes several components including plastics, organics, directly recyclable materials, and residual materials. The organics can be mainly or substantially or fully composed of paper and carton materials. The MSW feed stream is a relatively complex waste mixture, which can be obtained from a landfill or directly from waste collection vehicles, or supplied as a fraction of MSW in the form of e.g. RDF/SRF/DSD material according to availability. The general composition of the MSW feedstock can be determined or estimated based on, for example, the source of the MSW or feedstock sampling. An approximation of the composition of the MSW can be generated periodically or continuously in order to inform downstream unit operations. It is also noted that the MSW feed stream may include various different subtypes of plastics, organics, and the like, which can be leveraged to provide an adapted process and particular end products.

Still referring to Fig 1, the MSW feed stream 12 is subjected to separation 14 in order to produce different output streams that will be discussed below. In some implementations, the MSW feed stream 12 can be separated into a directly recyclable fraction 16, a plastics fraction 18, an organics fraction 20 and a residuals fraction 22 (also referred to as "rest"). It should be noted that various combinations of such separated fractions or fractions can be produced, and that not all of the fractions must be generated. In addition, the separation 14 can be performed using various equipment that can be operated in series or in parallel. It should also be noted that each fraction can include so-called impurities relative to that fraction, e.g., the plastics fraction 18 can include residual amounts of organic material or other non-plastic material.

The separation 14 can be performed by various solid separation and sorting units. The incoming MSW is thus separated into more homogeneous waste streams, as described above, which facilitates further processing. The MSW is composed of materials that have different physical and chemical properties, which can be leveraged to separate the feed stream of MSW into separate component groups. Examples of such differences are size, density, color, ferrous, non-ferrous, optical absorption or reflection of light properties, etc. One or more of such properties can be used to perform or control the separation. In addition, there may be an upstream monitoring step (not shown here) that detects and records information regarding the MSW feed, the composition of which can change over time. The properties that are monitored can then be used to control or adjust the separation step 14, or one or more units that are part of the separation step.

In one example, the separation installation 14 can include various equipment, such as wind and/or drum sifters to separate for size and density; magnetic and eddy current separators for separating ferrous and non-ferrous metals; optical sorters to separate different kinds of plastics (e.g., PET, PVC, PE/PP, PS); and shredders and/or washers to condition different fractions for either reuse (e.g., in the case of directly recyclable materials like directly recyclable PET, PVC, PE or PP) or further processing (e.g., plastics-to-fuel).

After separation 14, the directly recyclable fraction 16 can be further separated in a secondary separation step 24 in order to produce a ferro materials stream 26 and a non-ferro materials stream 28. Alternatively, the recyclable ferro materials stream 26 and non-ferro materials stream 28 can be obtained as part of the main separation 14. Additional post-treatment methods can also be implemented on the recyclable streams, such as washing, granulation/shredding, further purification, and so on. These recyclable materials can then be supplied to the market for such materials for reuse. Typically, these materials are not subjected to further processing beyond simple separation, although in some implementations additional processing is possible. For the separation of ferro materials, a magnetic separator, which can include an overband magnet, can be used. Non ferro materials can be separated using subsequent eddy-current separators dividing the stream further into subcategories (e.g., tetra packs and other non ferro materials). It should be noted that other ferro and non ferro separation devices can be used. Preferably, such a magnetic separator can be part of the main separation unit 14 as are the other elements of the separation step which produces output streams 18, 22, 20, 24 and 26.

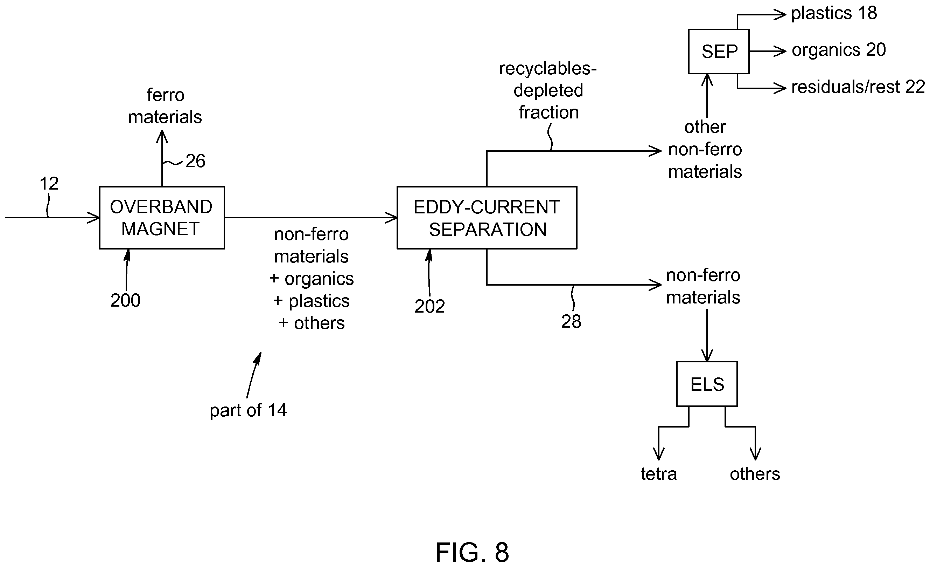

Referring to Fig 8, the separating of the ferro and non-ferro materials can include several sub-steps, including a magnetic separation step 100 to obtain the ferro materials stream 26, and an eddy-current separation step 102 for separating a non ferro materials stream 28 from the main stream. "Tetra" should be understood as referring to a packing material that is often used for liquids like milk and juices, and is typically layered material with aluminum foil, carton and plastic depending on the material and application (e.g., 4% Al foil, 74% carton, and 22% PE). The main stream from which recyclables have been removed can then be subjected to additional separation operations, as described herein.

Referring still to Fig 1, the different separated fractions 18, 20, 22 can each be subjected to further processing in order to produce end products or streams. In addition, the different processing of the fractions can be integrated together in various ways to enhance the overall operations, as will be described in further detail below.

### Plastics processing

The plastics fraction 18 can be processed in order to produce one or more plastic products, such as polyvinyl chloride (PVC), polyethylene terephthalate (PET), polyolefins that may be a blend of polyethylene and polypropylene (PE/PP blend) for example. Alternative or additional polymeric materials can also be made via various plastics processing techniques.

In some implementations, two separate plastics streams are produced by the separation unit 14, a rigid plastics fraction and a plastic foil fraction. The plastic foil fraction can be considered an end-of-life fraction, in part due to difficulty in sorting and cleaning. The rigid plastics fraction can be processed further to produce relatively pure PE, PP and PET streams that can be recycled.

In some implementations, as shown in Fig 1, the plastics fraction 18 is first separated into an end-of-life plastics stream 30 that is subjected to plastics-to-fuel (P2F) conversion 32, and a primary plastics stream that is subjected to plastics processing 34 to produce one or more plastic products. This initial plastics separation can occur as part of the main separation stage 14. The plastics-to-fuel conversion 32 can be performed by a P2F installation that converts plastics into diesel according to the EN590 norm, using various equipment and technical methodologies, such as those provided by BlueAlp™ (e.g., as disclosed in patent application No. WO2016116114, Weser, entitled "Method and system for transferring plastic waste into a fuel having properties of diesel/heating oil"). In some implementations, the plastics-to-fuel conversion 32 includes several sub-steps designed to obtain a target end product, such as a diesel equivalent fuel (e.g., EN590 certified diesel). In addition, paraffin and other hydrocarbons can be obtained as products for use as-is or for use as base materials in the petrochemical industry. For example, jet fuel could be produced and/or various other hydrocarbon end products.

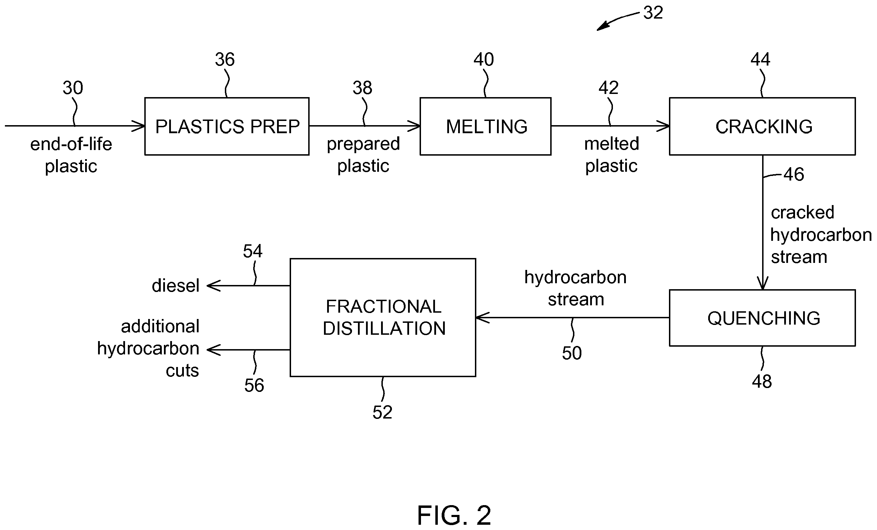

Referring now to Fig 2, the plastics-to-fuel conversion 32 can include various sub-steps and units to produce end products. The end-of-life plastics stream 30 can be supplied to a plastics preparation unit 36 to produce prepared plastic 38 which is sent to a melting unit 40 (e.g., in an oxygen-free environment) to produce melted plastic 42. The melted plastic 42 can then be supplied to a cracking unit 44 to facilitate breakdown of longer hydrocarbon chains into shorter cracked hydrocarbons, thereby producing a cracked hydrocarbon stream 46. The cracking unit 44 can be operated using various methodologies that utilise heat, pressure and/or catalysts to facilitate cracking reactions. The cracking unit 44 can be configured in accordance with the particular plastics to be processed, and the required or desired outputs or operating conditions, although preferably no catalysts are used. In addition, the cracking reactions can also produce light hydrocarbons (gas and liquid) and char, which can be recovered. Waste gases and light fractions can also be reused to generate energy that is used in the plastics-to-fuel conversion 32 installation and/or various units in the overall process. Such light hydrocarbon (LHC) fraction can be recovered from at least a downstream distillation unit. Waste gases include non-condensable gases (NCG) which can be cleaned in a gas washer and then used in the thermal oil boiler together with part of the light hydrocarbon fraction, with the remaining part of the light hydrocarbon fraction being used to power a gas motor to produce electricity.

In some implementations, the cracking reaction can be temperature driven and can include a random factor. The aim of the cracking step is to form a diesel pattern of hydrocarbons, *i*.*e*., a distribution of hydrocarbons having carbon length and properties such that a notable fraction is suitable as diesel fuel, although there is some formation of lighter and smaller carbon chains (LHC and NCG). In generals, LHC are liquid and NCG are gaseous. The bulk of these products are formed during the cracking reactions, although some additional formation can occur in downstream steps (e.g., distillation) that may be operated at elevated temperatures. Heating during the following unit operations (e.g., distillation) can entrain some cracking of longer chain products into smaller ones.

The cracked hydrocarbon stream 46 is supplied to a quenching unit 48 which cools the stream, preferably by rapid and controlled cooling to stabilize the cracked hydrocarbons and obtain the desired chemical properties of the resulting hydrocarbon stream 50. The hydrocarbon stream 50 can then be supplied to a fractional distillation unit or another type of separation apparatus to obtain a purified version of the fuel, for example a diesel stream 54 and potentially additional hydrocarbon cuts 56.

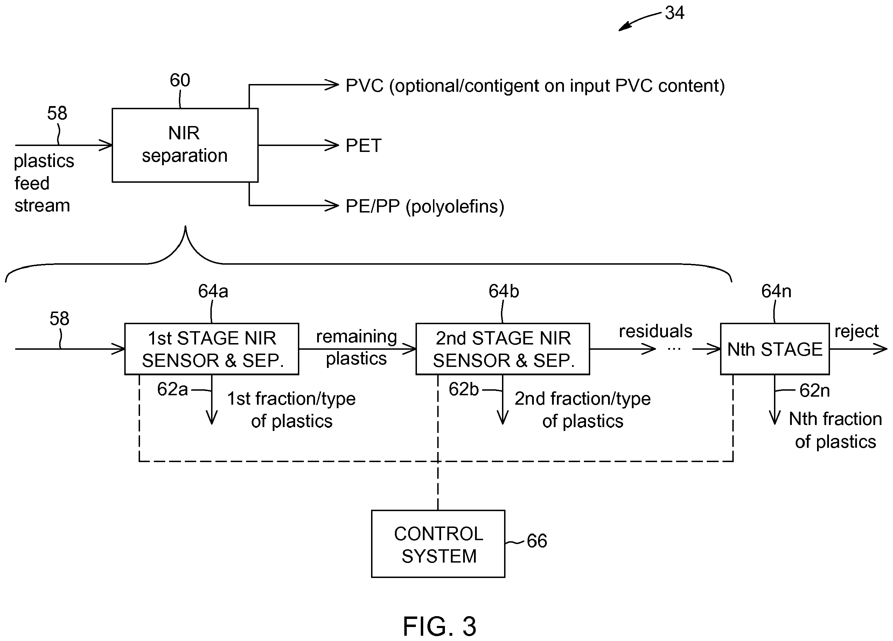

Turning back to Fig 1, the other portion of the plastics fraction 18 can be subjected to plastics processing to produce polymer products. Fig 3 illustrates a plastics feed stream 58 derived from the plastics fraction 18 (shown in Fig 1) that is supplied to plastics processing 34. It is noted that that the plastics feed stream 58 can be the same stream as the plastics fraction 18, when no intervening processing or separation is performed on the plastics fraction 18. There can also be optional removal steps to remove undesirable plastics for the plastics-to-fuel process (e.g., PET, PVC). As shown in Fig 3, the plastics processing installation can include a main separation unit 60 which can include a near infrared (NIR) separation capabilities that use NIR spectroscopy to facilitate identification of different types of plastics so that proper sorting can be done. NIR spectroscopy can enable identification and sorting of different polymer types including PET, PS, PP, PE and others. NIR sensors can be incorporated into a system in various ways. For example, referring to Fig 3, there can be multiple stages for sensing and separation, where each stage identifies and removes a predetermined category of plastics based on the measured NIR spectral data, thus producing recovered plastics streams 62a, 62b, 62n, and corresponding remainder streams for further processing or eventual rejection. Each NIR sensor and separation stage 64a, 64b, 64n is equipped with appropriate NIR sensors that are operated and calibrated according to certain NIR wavelengths and calibration curves, as well as equipment for removing the identified plastics from each stage. The sensors of each stage can be pre-calibrated to use different wavelengths. In addition, the sensors and the other separation equipment can be connected to a control system 66 that facilitates the operation of this plastics processing method.

Regarding the plastics processing operation, plastic polymers can be identified by their absorbance patterns using NIR techniques, particularly in the range of about 700 to 2500 nm. Using two wavelengths (e.g., 1656 nm and 1724 nm), the relative reflectance of the plastic identifies chemical composition and allows the selective separation of the plastic waste stream. Depending on the plastics to recover, different NIR-based techniques can be used.

It is also noted that in some implementations, the NIR separation is performed to recover separate streams of PVC, PET, and PE/PP. Nevertheless, it is also possible to perform the NIR separation to recover only one or two of these plastics streams (e.g., only PE/PP) while the other plastics streams (e.g., including PVC and PET) are supplied to the subsequent unit operations. It is also possible for the process to be regulated such that the type and amount of each plastic can be recovered or subjected to plastics-to-oil conversion at different times. This regulation can be controlled based on requirements of the plastics-to-oil conversion units, the composition of the plastics fraction 18, the operating capacity of the NIR separation units, and/or the value of the different output products (e.g., diesel versus recycled PVC, PET, and PE/PP).

It should also be noted that various other separation techniques can be deployed in the context of plastics separation and processing, such as shredding followed by separation by sink-float methods to enhance purity levels; friction cleaning; and friction drying.

### Organics processing

Turning back to Fig 1, the organics fraction 20 or at least a portion thereof can be converted into second generation biofuel and/or bio-products. The particular treatment and unit operations that are used to process the organics fraction 20 may depend on the composition and flow rates of the fraction. In some implementations, the organics fraction 20 is mainly composed of cellulosic material and can be treated accordingly. The processing of the organics fraction can be referred to in general as second-generation bio-product production 70.

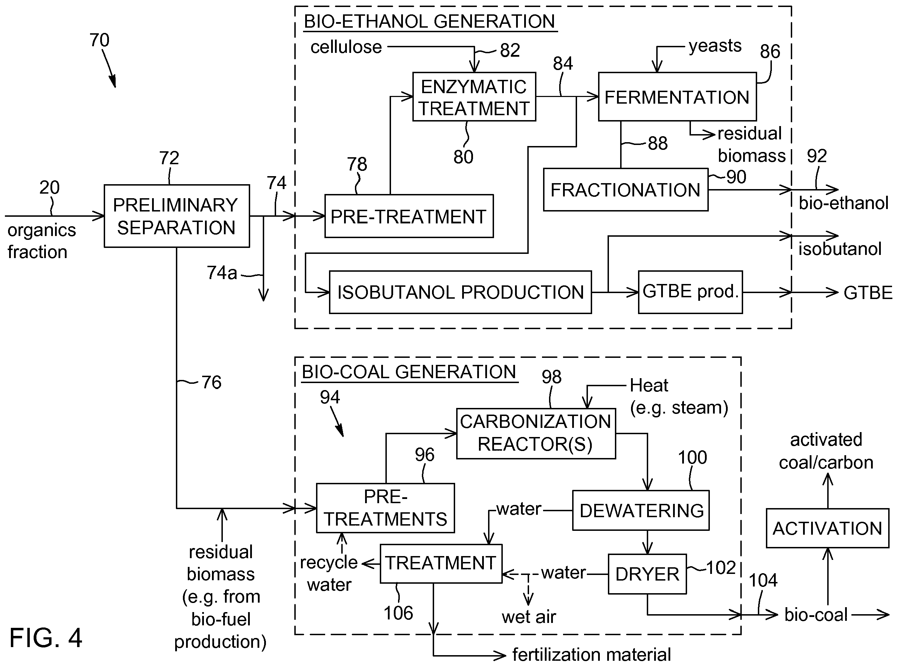

Referring to Fig 4, the organics fraction 20 that includes cellulosic material can be subjected to various unit operations in order to produce desired bio-products. The organics fraction 20 can be subjected to a preliminary separation 72 to produce a first organics stream 74 dedicated to liquid fuel production (e.g., bio-ethanol) and a second organics stream 76 dedicated for solid bio-product production (e.g., bio-coal). Other organics streams can be isolated or separated and then treated in order to produce other bio-product streams.

In some implementations, the organic streams 74 and 76 are generally the same, and thus constitute a split main organics stream. Alternatively, the two streams can be different due to an upstream separation of some kind in order to tailor each stream to the downstream processing. For example, initially stream 74 can be viewed as a clean paper and carton fraction that will be destined for recycling, but can be diverted in part or in whole for bio-ethanol generation. In that sense, part of the organics fraction 20 can be separated out for direct recycling (stream 74a), and such an organics fraction can be fed to direct recycling and/or bio-ethanol generation.

In some implementations, the first organics stream 74 can be subjected to pre-treatment 78 which can include a chemical treatment (e.g., acidic or alkaline environment, optionally under high pressure and/or temperature conditions such as autoclave), followed by an enzymatic treatment 80 using cellulase 82 to convert the cellulose into sugars to produce a sugar-enriched solution 84. The sugar-enriched solution 84 can then be subjected to fermentation 86 to convert the sugars into alcohols, e.g., to produce a bio-ethanol containing solution 88. Residues from the fermentation process can also be removed and reused. Subsequently, the bio-ethanol containing solution 88 can be subjected to fractionation 90 to produce second-generation bio-ethanol product 92. Various cellulose-to-ethanol methods and units are available and can be used in the context of this bio-ethanol production step. In addition, various yeast strains can be used and developed to be adapted for efficient and effective conversion of sugars to ethanol and isobutanol at the desired conditions of operation.

Still referring to Fig 4, a portion of the sugar-enriched solution 84 can be diverted (as a slip stream continuously or as part of or all of the stream 84 when not being fed to fermentation 84 for ethanol production) for the production of isobutanol. Isobutanol production can also involve fermentation, using different yeast strains for example, and fractionation/distillation. Bio-isobutanol can be added as such to gasoline or diesel, or can be processed for producing glycerol tertiary butyl ether (GTBE) using various techniques. In the case of producing GTBE, isobutanol can be converted using an acidic catalyst of glycerol (by-product of biodiesel production) with isobutene (obtained via dehydration of the produced bio-isobutanol). Various GTBE production methods can be used. GTBE can be used as a fuel additive to both diesel and gasoline to improve performance and reduce soot emissions. GTBE can be produced via a catalysed reaction of glycerol and isobutene.

The second organics stream 76 can be converted into bio-coal. The stream 76 can be combined with at least a portion of the residual biomass from the fermentation 86 or other unit operations of the bio-ethanol generation, such as GTBE production, and then converted into bio-coal pellets. The bio-coal production process can include hydrothermal carbonization (HTC) and can include units and process features of Ingelia™, for example. For instance, methods and systems described in EP2719748, Hitzl et al., entitled "Method for extracting biochemical products obtained from a process of hydrothermal carbonization of biomass", or EP2366757, Hitzl, entitled "Pressure and temperature control system for at least one chemical reactor", can be used. The hydrothermal carbonization enables wet biomass in a slightly acidic environment and at high temperatures and pressures to generate hydrophobic bio-coal molecules. One feature of the process can be that it takes place in an aqueous environment, meaning that humidity of the biomass is not a problem. In addition, there are no toxic waste products that are generated during the process and the process water contains substantially only soluble components such as N, K and Fe, which can have a fertilizing effect on plant growth. In addition, due to exothermic reactions taking place the thermal energy consumption is relatively low, and is mainly involved for start-up operations. The hydrothermal carbonization process produces bio-coal which concentrates almost all of the carbon present in the original biomass.

The bio-coal is a solid biofuel with a similar energy content and physical property profile as naturel coal, but bio-coal burns cleaner. The bio-coal can be used as a drop-in fuel in coal fired power plants as a carbon neutral fuel.

In some implementations, the hydrothermal carbonization 94 can include a number of sub-steps and units, such as pre-treatment 96 (e.g., heating, mixing, etc.), carbonization reactors 98 (e.g., single or multiple stages arranged in parallel or in series and operated optionally a different operating conditions), a dewatering unit 100 (e.g., filter press or the like), a dryer 102 that produces the bio-coal product 104 (e.g., which can be in various forms according to a post-drying treatment to produce powder, granules, pellets, briquettes, etc.). There can also be a treatment unit 106 for receiving and processing the aqueous solution from the dewatering unit and the dryer, in order to produce various streams such as a solids-depleted water stream, a water stream suitable for steam generation, a solids-enriched material that can be suitable for fertilization applications, and so on. In addition, the process water can be reused internally in the process to maintain the acidic environment, where appropriate. Overflow/blowdown can be treated in the central waste water treatment facility (e.g., evaporator).

Alternative processing approaches can be used instead of or in addition to hydrothermal carbonization. For example, in some implementations, incineration, pyrolysis or gasification could be used. Each of these processing alternatives have positive and negative points, and it is noted that hydrothermal carbonization can be the preferred option in the present context.

An alternative may include a kiln (e.g., rotating gasification unit) in which the process temperature may be kept between 450-500°C. To facilitate effective gasification in the kiln, the kiln can be equipped with a recycling of corundum (an aluminum oxide based material) grains. This material is used as heat transfer medium to the material which is processed in the kiln. Because of the high temperature of the corundum material and the rotating effect, the materials which are to be processed are heated and gasified in an effective manner. By heating the materials, gasification can be optimized, and the rotating kiln can facilitate prevention of solidification of the materials which are to be gasified. It is also noted that materials other than corundum can be used to provide the heat transfer grains.

The gasses leave the kiln at a temperature between the 450-500°C and can then be mixed with air in order to facilitate complete combustion at a temperature of about 950°C with a residence time of about 2 seconds, for example. By operation at this temperature and residence time, formation of dioxins is inhibited and low NOx output of the flue gasses can be encouraged.

Once the gasification of the materials is completed, the remaining inert materials (e.g., ashes, inorganic materials, etc.) can be filtered from the grains and the grains can be reused in the gasification process. The filtered materials can be used, for example, as filler as indicated elsewhere in the present disclosure.

### Residuals processing

Referring to Fig 1, the residuals stream 22 can be fed to a thermal reactor 108 for processing into inert material 110, which can be used in various applications such as filler for the construction industry. An example of the thermal reactor can be referred to herein as a scavenger unit. The thermal reactor 108 can also receive other by-product streams from the plastics processing 34 and/or the biofuel production 70. The thermal reactor can include a fluidized bed type reactor (e.g., a fluid sand bed reactor FSBR) for conversion of waste residue to an inert state. The FSBR can be, for example, one provided by Arena Comet™ or Petrogas™, or other types of reactors. The FSBR facilitates conversion of the residuals 22 into a COPRO-certified (or other certification standard or requirement) material that is inert and can be used as filler in various products (e.g., concrete tiles, bricks, etc.).

In some implementations, the FSBR is be based on the Unecco™ conversion process, which is a physicochemical and catalytic conversion process characterized by very high surface area contact between fluids and solids per unit bed volume, high relative velocities between the fluids and dispersed solid phase, high levels of intermixing of the particulate phase, rapid mass transfer between the fluid and the solid phase, frequent particle-to-particle and particle-to-wall collisions, and released energy that can be recovered to power other units in the overall process. Some examples of alternative processes for treating the waste residues are plasma-torch processing or incineration.

In some implementations, the thermal reactor be a fluidized sand bed or similar reactor through which air is blown vertically from bottom to top at a speed that keeps the sand particles, or similar material, floating and suspended and thus enables the sand to behave like a hot liquid. The material to be processed is introduced into the reactor just above or directly into the bed (e.g., via pumps or screws). The system can respond rapidly to changes in load demand due to quickly establishing thermal equilibrium between the air and the particles in the bed. The operation of the thermal reactor (e.g., FSBR) at relatively lower temperatures can help reducing air pollution. The low temperatures operation also reduces the formation of nitrogen oxides. In addition, by adding lime stone (calcium carbonate) to the reactor the discharge of sulphur dioxides to the atmosphere can be neutralized. It should be noted that lime addition is optional for various parts of the process. For example, in one implementation, there can be no lime addition in the thermal reactor (e.g., FSBR) and there can be lime addition in the general flue gas cleaning in the process.

In some implementations, the material fed to the thermal reactor (e.g., FSBR) is between 40-45 wt% dry content depending on the caloric value of the material. The material fed to the thermal reactor (e.g., FSBR) is, in some implementations, a sludge stream, such as e.g., waste water treatment sludge.

### Mass balance implementations

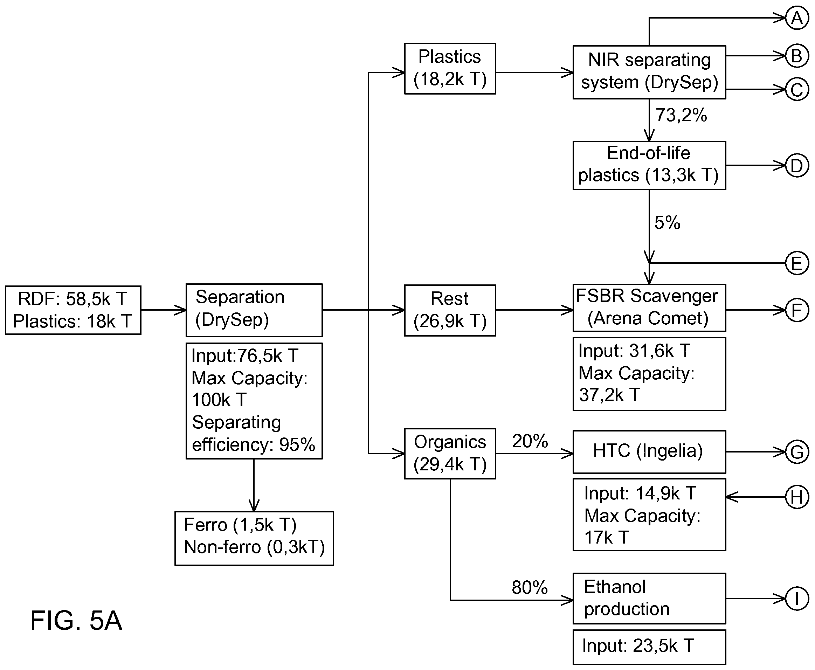

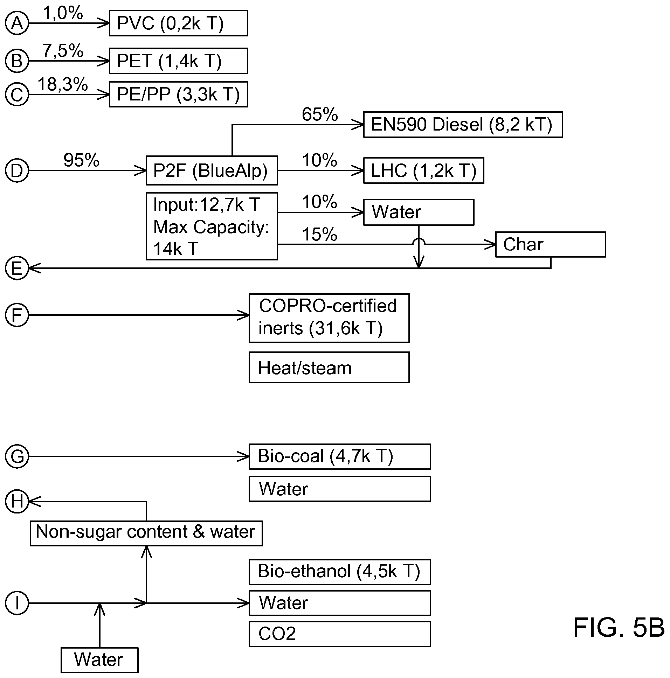

Referring to Fig 5, an example mass balance is shown for an implementation of the overall process.

Pursuant to this method, approximately 95% or higher (e.g., 99%) of the waste that is input into the process is given a useful purpose in terms of marketable end-products, recycling, energy generation to produce power for the process for example, reintroduction of waste water into the process, and so on. In addition, 57% of energy in the waste can be recovered in this example. Notably, the integrated processing facilitates self-sustainability in terms of energy consumption, as heat and electric power can be recovered from the process and used throughout the process.

Substantially all of the CO₂ can be captured and neutralized. CO₂ can be purified and stored for eventual sale, for example for use in a greenhouse project that can be, for example, proximate to the MSW treatment installation or remote.

In addition, waste water is treated, cleaned and can be reintroduced into the process. GTBE production is not included in the attached mass balance but could be incorporated.

Of course, it should be understood that the mass balance illustrated in Fig 5 is not meant to limit the scope of the technology described herein, and should be viewed as an example of the integrated process for treating MSW with example masses and volumes of the various streams, some of which are also optional.

It is also noted that the mass balance can be modified over time and in conjunction with the deployment of different parts of the overall system. For example, in some scenarios, it may be advantageous to deploy the plastics processing, plastics-to-oil conversion, and scavenging parts of the process as part of a first stage, and then deploy the bio-product production part of the process at a later date as part of a second stage. In the context of this optional modular deployment, the mass balance can be modified such that the first stage supplies part or all of the organics fraction to bio-coal generation, storage, disposal, and/or as part of the fraction that is fed to the thermal reactor (e.g., FSBR). Other deployment and roll-out methods can also be implemented. In one example, during the first stage the organics fraction is fed to the bio-coal generation facility, only during the second stage is the organics fraction separated and fed into both the bio-ethanol and bio-coal generation facilities.

In addition, if energy balance of the facility comes out such that there will be energy needed from outside (grid), the choice can be made to use some produced material (e.g., diesel-fuel, bio-coal, ethanol, or any combination thereof) to produce energy to power the facility. However, if local situation dictates, the choice can be made to maximize waste treatment capacity and produce maximum products and thus use grid power. The facility can also include a solar park, on the roof or not, a windmill, etc., to power (part) of the facility. The choice could be made to operate the feedstock sorting and conditioning part of the facility only when e.g. during daylight hours when sunlight is available.

### Integration techniques

The process for treating MSW can include various integration features where heat and/or mass is advantageously leveraged by integrating between different parts of the process. Fig 1 schematically illustrates heat and/or mass integration (HeMal) between the plastics processing operation 34, the thermal reactor operation 108 and the bio-product production operation 70.

In terms of heat integration techniques, a heat recovery system 114 can be provided and can be an organic Rankin cycle (ORC) based system. The heat recovery system 114 can be integrated into the overall process to recover heat released by various unit operations. A suitable organic fluid can be used as the heat transfer fluid, e.g., silicon-based, refrigerant-based, hydrocarbon-based, etc., depending on the application. The heat transfer fluid is confined to a closed and leak-free circuit and can be pre-heated and vaporized using the heat source in a preheater and evaporator. The heat transfer fluid expands in a turbine, which is directly coupled to an electric generator, and is condensed using a closed water loop in a shell-and-tube heat exchanger, for example. Alternatively, ambient air could be used for cooling purposes. The condensate can be pumped back to the evaporator, thus closing the thermodynamic cycle.

Application of such ORC systems in the context of the overall process can present a number of advantages. The ORC system has a high efficiency, low mechanical turbine stress, low RPM of turbine allowing direct drive of the electric generator without gear reduction, high cycle efficiency, long life due to absence of moisture that causes erosion of turbine, low OPEX, good performance with partial load, high reliability, availability and low noise operation, autonomous operation, and so on. In addition, it should be noted that the overall process and individual unit operations thereof can be provided such that in the remote possibly that the ORC unit goes offline or fails, the operation of the primary process would not be affected. In some implementations, flue gases from the different unit operations are recovered and centralized so that energy can be recovered via the ORC. For example, flue gas streams can be supplied to a central energy recovery unit that is part of the ORC in order to transfer heat from the flue gases to the heat transfer fluid.

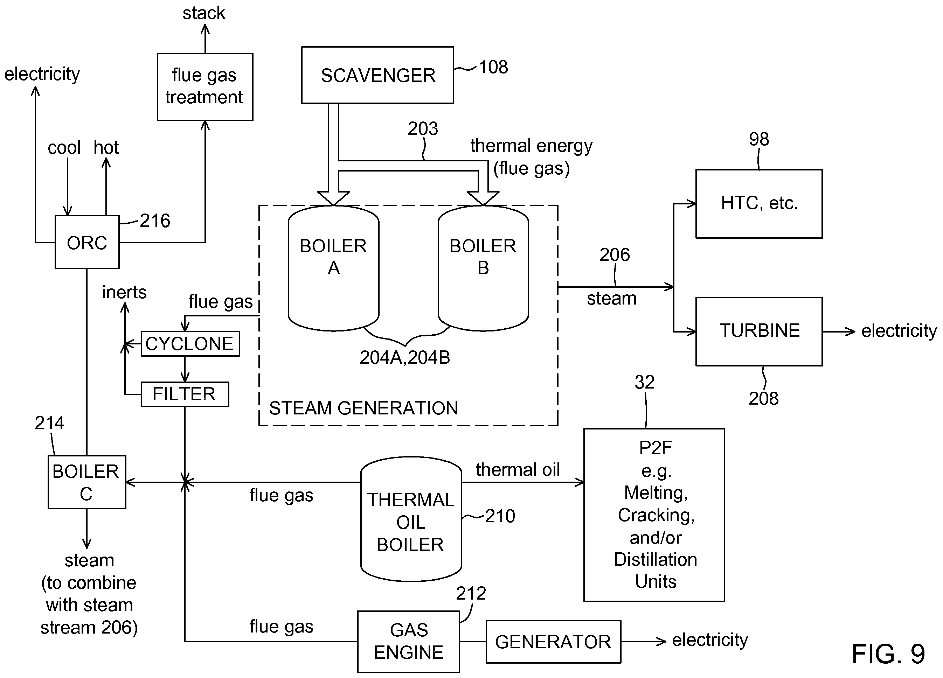

Energy (heat and steam) can be recovered from various units for reuse, e.g., to produce electricity to power various parts of the plant. For example, referring to Fig 9, thermal energy 203 from the thermal reactor 108 can be used to produce steam in two steam boilers 204A, 204B. This steam 206 can be used partially to fuel other processes (e.g., HTC, shown as 98 in Fig 9), and partially to power a steam turbine 208 generating electricity. The exiting flue gasses can then be mixed with the flue gasses from a thermal oil boiler 210 of the P2F process and the flue gasses from a gas turbine or reciprocating engine 212 (which powers a generator producing electricity to power part of the rest of the plant). This stream can fuel a third steam boiler 214 (boiler C) and subsequently an ORC unit 216. The steam production from boiler C can be combined with the steam from the first two boilers 204A, 204B to provide heat for other processes and power the steam turbine 208.

The cycle of energy production as electricity, followed by the heat recuperation from the engine as well as from exhaust gases, can be referred to as combined heat and power (CHP). The principle can be adapted to the cycle of technologies and unit employed in the present process, so that power is produced and heat is recovered using a multistep methodology until the heat is economically depleted (i.e., heat level to low). For example, high energy flue gases can be fed to steam generation units such additional energy is removed from the flue gases in progressive fashion via each subsequent step. As a final step, the flue gas can be fed to the ORC.

Various by-products of applied unit operations of the overall process can be used as fuel for energy producing machines (e.g., gas engine, gas turbine), and the resulting exhaust gases can be harvested as high caloric streams and used to help produce steam. This steam can be applied in heating other processes of the overall process and/or as driving medium for steam turbines that produce electricity. Condensates are recovered and re-used in steam production.

The cooled exhaust gases are used to heat a recuperative loop, feeding thermal energy to the ORC to heat the heat exchange fluid. This ORC produces electric energy, provided a sufficient cooling medium is present. This can be running water or air cooling.

Various mass integration techniques can also be used in the context of the overall process, where streams from certain sub-processes and fed into others.

For example, by-products considered as waste for the separate sub-processes can be fed into the thermal reactor / scavenging unit (e.g., FSBR). If any caloric value is present in a given stream, it can contribute to the process heat of the thermal reactor. The resulting flue gases from the thermal reactor (e.g., FSBR) carry thermal energy and exchange these to steam in the thermal reactor steam boilers. The steam flows can also be grouped and used throughout the processes.

In addition, material streams can be recovered from certain units and advantageously used in others. In particular, high caloric rest products from the P2F (also referred to as P2O for plastics-to-oil) and HTC processes can be processed by the thermal reactor yielding a higher energy output, which can be reused as described above. Organic material that cannot be effectively converted to bio-ethanol can be processed by HTC technology to produce additional bio-coal.

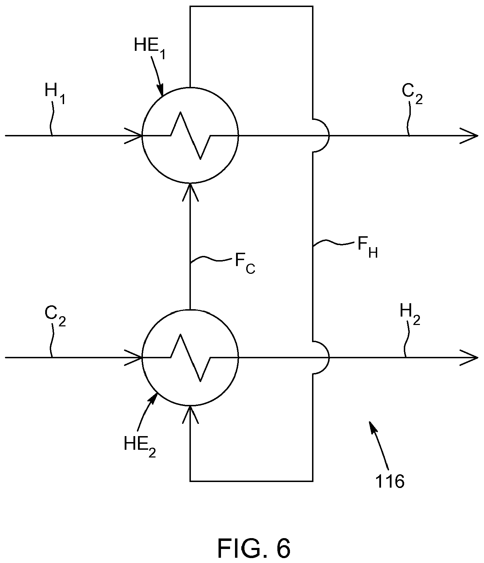

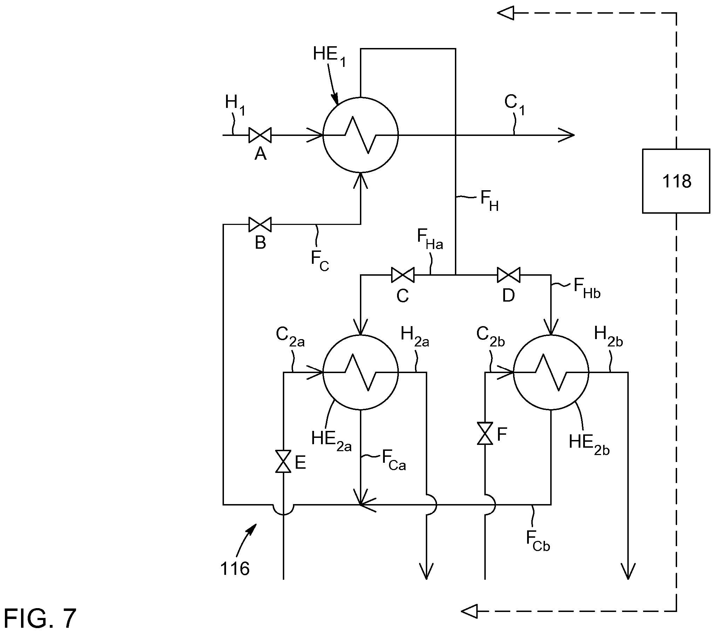

Referring to Figs 6 and 7, a heat exchange system 116 can be provided in order to recover heat and transfer it to another part of the process. For example, as shown in Fig 6, the heat exchange system 116 can be configured to recover heat from a hot process fluid (H1) and transfer at least a portion of that heat to a cool process fluid (C2), thus producing a cooled process fluid (C1) and a heated process fluid (H2). Indirect heat exchangers (HE1, HE2) and a heat transfer fluid (F) can be provided for this purpose, as illustrated. The heat transfer fluid is initially cool (i.e., FC), is heated in the heat exchanger (HE1) to produce a heated transfer fluid (FH) which is then used in the other heat exchanger (HE2) to heat the other process fluid.

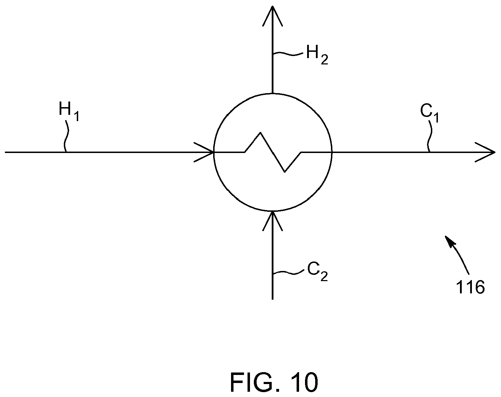

As an example, the HTC process can exchange heat from the output mass to the feed mass by circulating a heat exchange fluid (which can also be called a heat carrier). The heat carrier can be heated in the heat exchangers of the final product out of the reactor, and heat in another heat exchanger the feed mass going to the reactor. Thus, H1 would be the hot output of the HTC and C2 would be the cool input (e.g., see Fig 10). Rather than having a heat exchange fluid that indirectly transfers the heat between two streams, it is also possible for H1 and C1 to pass through an indirect heat exchanger in order to transfer heat therebetween. Additional examples of heat exchanger use are the following: heat recuperation for the production of steam from the thermal reactor flue gasses; heat recuperation on the combined flue gasses stream for steam production and for ORC operation; heat recuperation from P2F (e.g., cooling at exit of cracking step). Once heat is recovered, it can be supplied to one or more other units of the overall process.

Referring now to Fig 7, the heat exchange system 116 can include multiple indirect heat exchangers (HE2a, HE2b) that receive different portions (FHa, FHb) of the heated fluid (FH) in order to heat two different process fluids (C2a, C2b) and produced two heated process fluids (H2a, H2b). The heat transfer fluid portions (FHa, FHb) are thus cooled to produce corresponding cooled transfer fluid portions (FCa, FCb), which can be recombined together to form a combined transfer fluid (FC) that is fed back into the heat exchanger (HE1) for heating. In this setup, where the heat transfer fluid is split into two or more portions for use in two different heating applications, there may be a heat transfer control system 118 which is coupled to various instrumentation and/or control valves (A to F) in order to control the heat transfer between the fluids.

For example, multiple heat carrier streams can be found in the possible interaction between HTC and P2F. Both can have a heat carrier loop, on different temperature levels. However, heat can be recuperated from waste streams (e.g., P2F residue) and used in the HTC loops. Additional heat can also be added from the higher temperature carrier loops. As an example, P2F residue cooling can heat the waste water feed to water evaporator (e.g., using the cooling circuit of the gas engine), and also the dryer loop in HTC.

It should also be noted that one or more heat exchangers can be used for HE1, HE2, HE2a, HE2b, and when multiple heat exchangers are used they can be provided as a bank of exchangers. The indirect heat exchangers can have various different constructions, e.g., shell-and-tube, plate type, and so on, depending on the desired application and heat transfer requirements. In addition, some alternative configurations can use multiple heat exchangers that recover heat from two or more different hot process streams, and the heated transfer fluid (which may be a single stream or multiple streams) can be reused to heat one or more cool process streams. The heat transfer fluid can also be used to recover heat sequentially or in parallel from two or more hot process streams, and then provide the heat to two or more cool process streams sequentially or in parallel. Thus, a heat transfer fluid and associated system and equipment can be used to recover heat from one or more hot process streams, and provide that recovered heat to one or more cool process streams in order to provide advantageous performance, heat reuse, and energy utilization.

In addition, there may be one or more heat exchange systems 116 having similar or different configurations to perform the desired heat recovery and reuse. In one example, a first heat exchange system can be configured to recover waste heat or low-quality heat and then provide such low-quality heat to one or more other units that can benefit from that type of heating. A second heat exchange system can also be provided and configured to recover high-quality heat to be reused appropriately in another part of the process.

Depending on the stream, multi-stage heat recuperation can be applied, e.g., heat recuperation from flue gasses can first be used to produce steam (e.g., can be considered "high quality" heat recovery), and afterwards this stream can be passed to the heat exchanger of the ORC unit to further extract heat. Since and ORC unit can run on lower input temperature, this can be considered a "low quality" heat.

### Optional deployment implementations

The coupling and synchronizing of the multiple units and sub-processes can be provided to enable advantageous operations. In addition to the above-mentioned integration techniques, a modular deployment strategy can be used.

For example, referring to Fig 1, the process can be deployed in stages including a first stage that includes deployment of the separation step 14, the plastics-to-oil conversion (P2F) step 32, the plastics processing to recover recyclable plastics 34 including at least one of PVC, PET and PE/PP, the scavenging step 108, and part of the bio-product generation step 70 which is preferably the bio-coal generation (shown in Fig 4). Thus, in the first stage, the bio-ethanol generation process is not implemented. Then, as part of a second stage, the bio-ethanol generation facility can be implemented, as well as any necessary modifications to the overall process, such as energy recovery and reuse infrastructure, separation units (e.g., unit 72 in Fig 4), instrumentation and control units, storage units for end products and intermediates, and so on. In addition, the second stage can exclude isobutanol and GTBE production, one or both of which being implemented at part of third or fourth stages of deployment.

Various other deployment strategies can also be implemented. In some scenarios, advantageous deployment can include deploying in the first stage at least one unit operation that can process each of the main separated streams 18, 20 and 22, and in the second stage at least one additional unit for treating a sub-fraction of one of more of these streams 18, 20, 22. In another example, only one of units 34 or 32 is deployed for the first stage, and the other is deployed during a second deployment stage.

It is noted that the setup of the installation can be done in stages with add-on units being implemented over time. For example, a primary process configuration could be constructed and operated without certain unit operations (e.g., without bio-ethanol, bio-isobutanol or GTBE production from isobutanol) with the GTBE and other production equipment being added on at a later date.

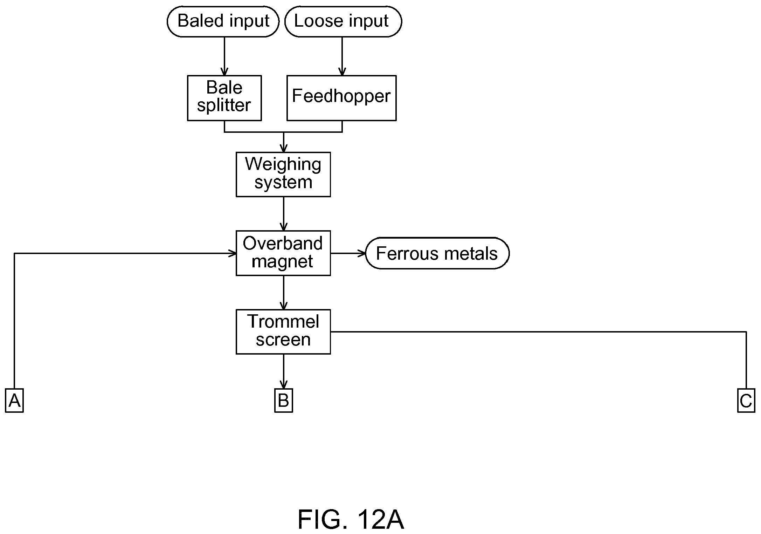

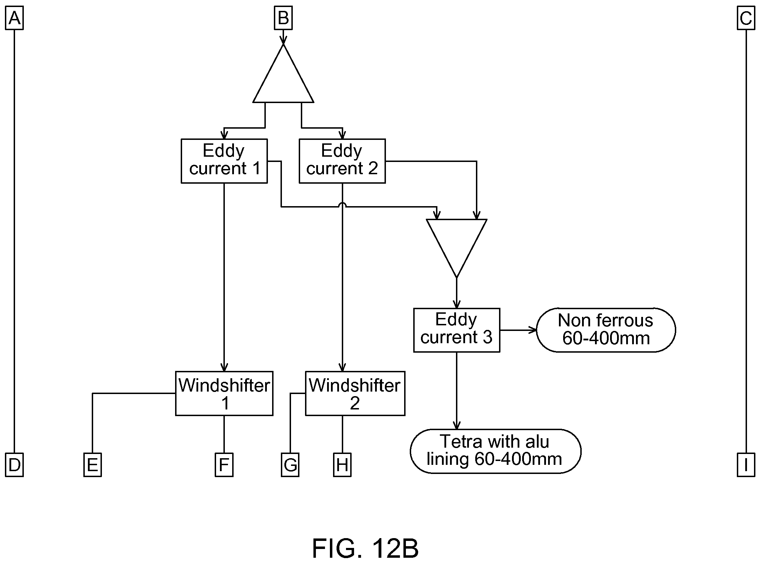

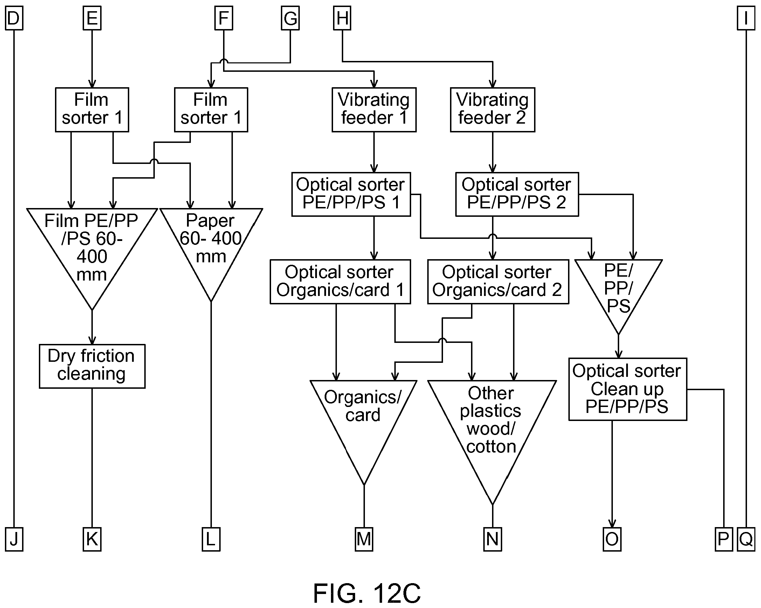

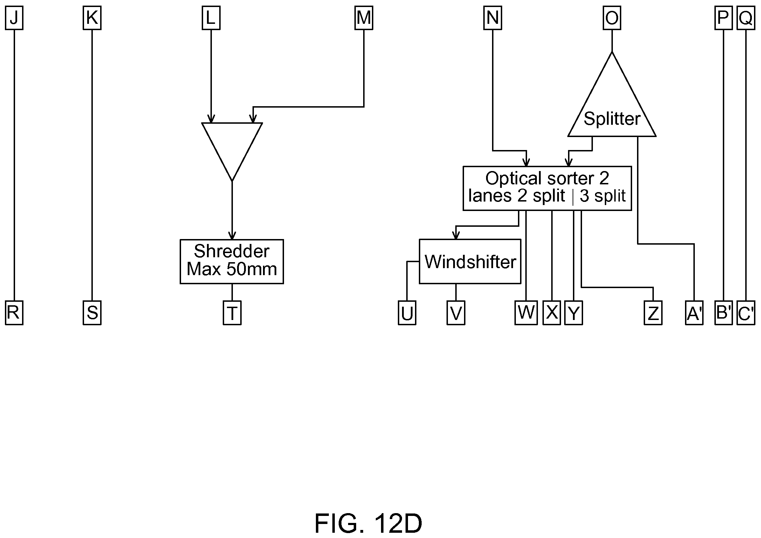

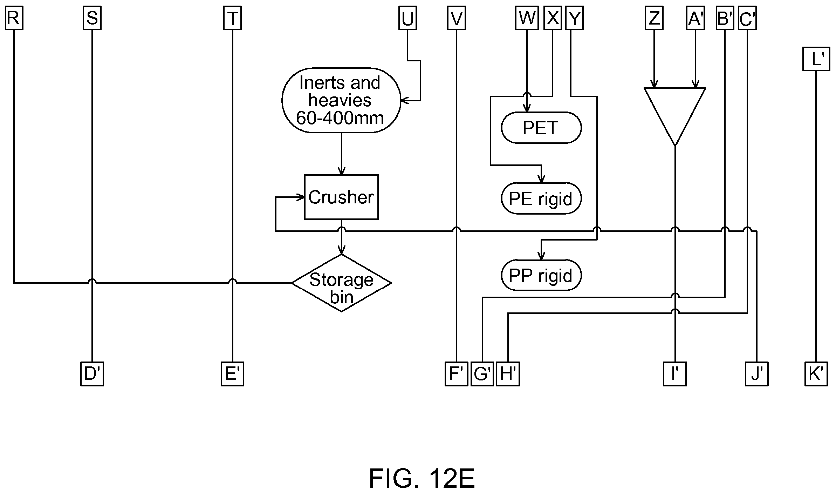

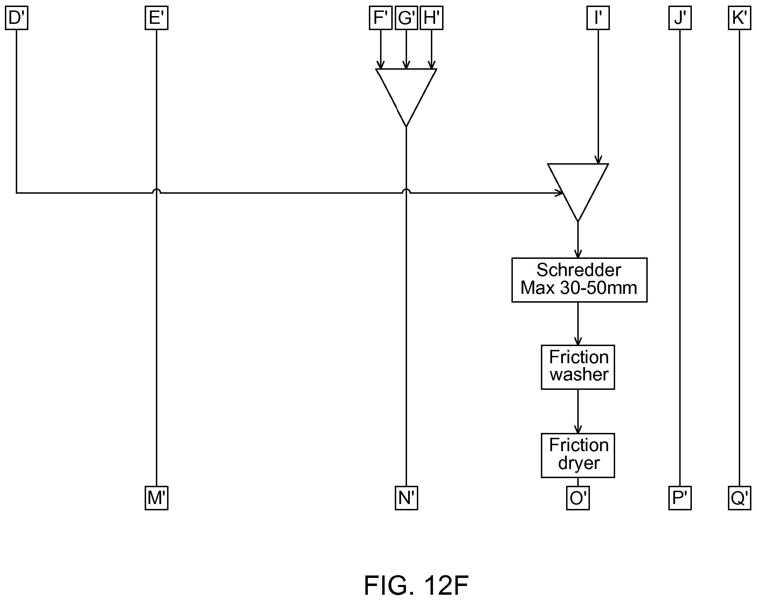

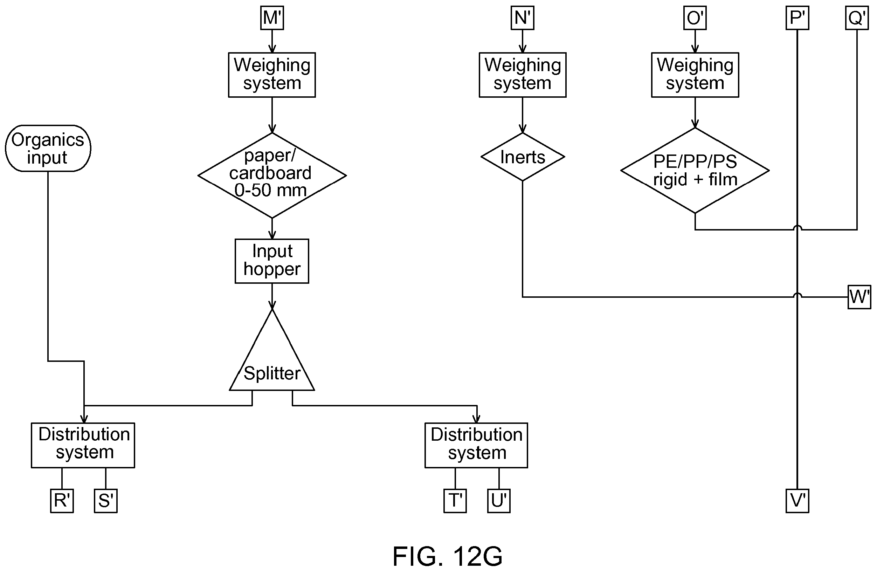

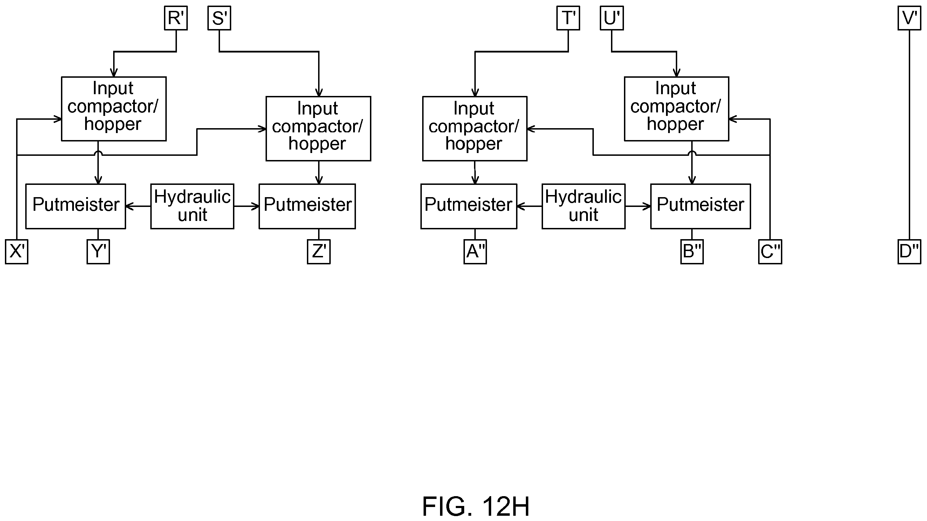

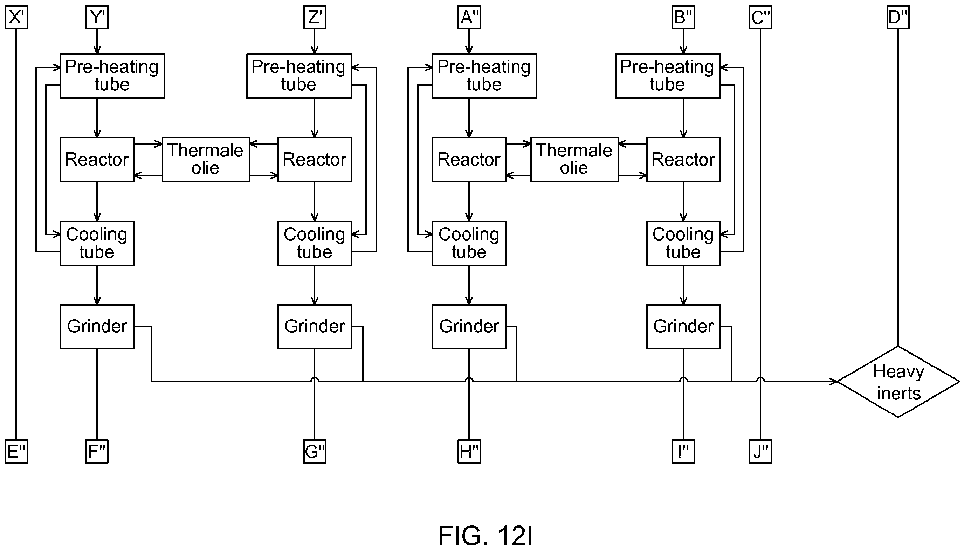

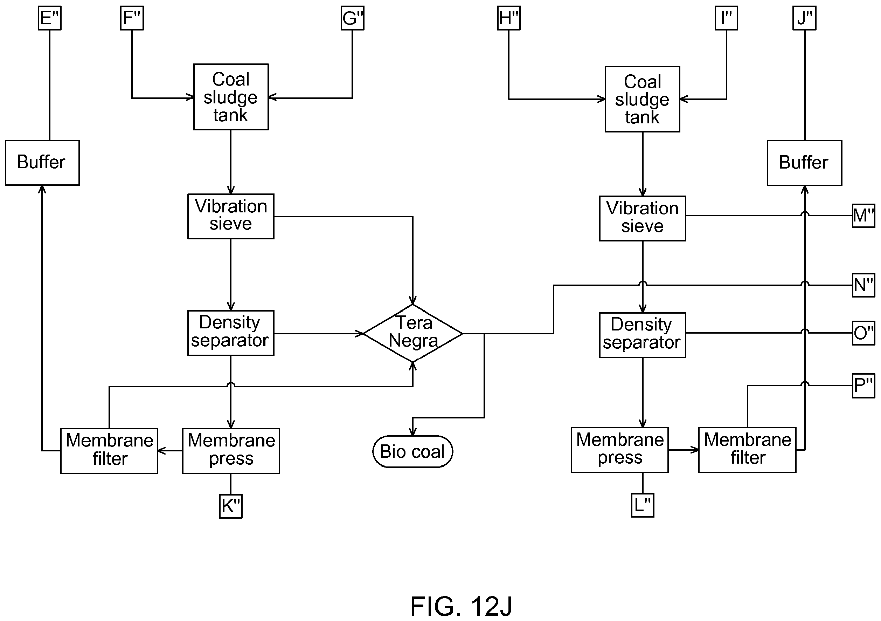

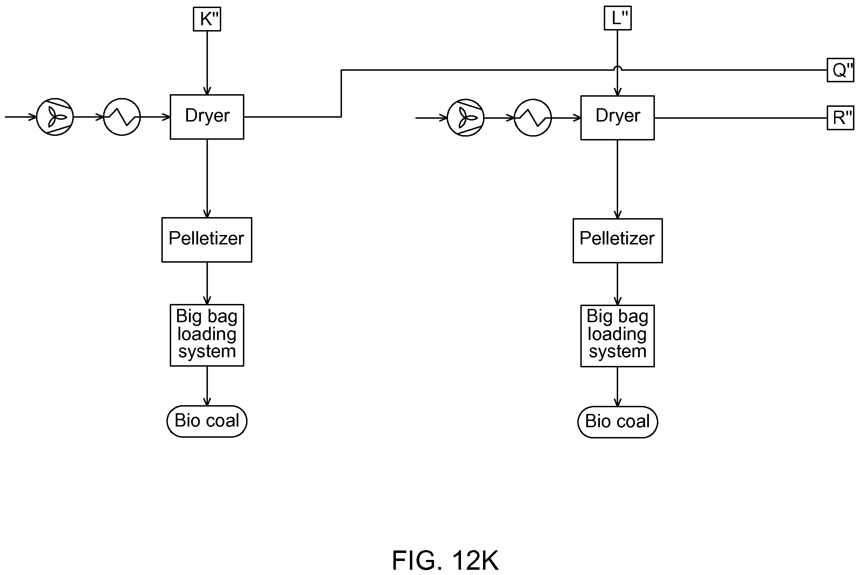

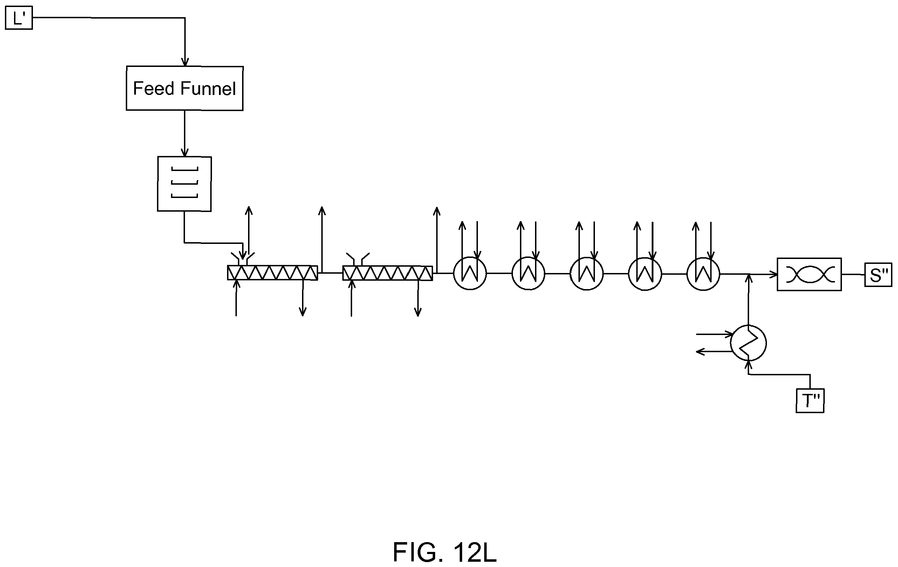

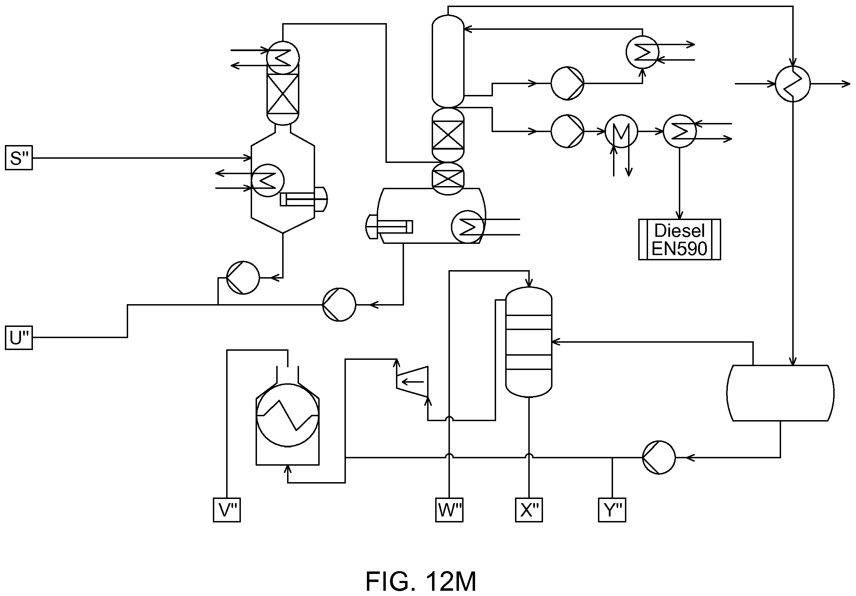

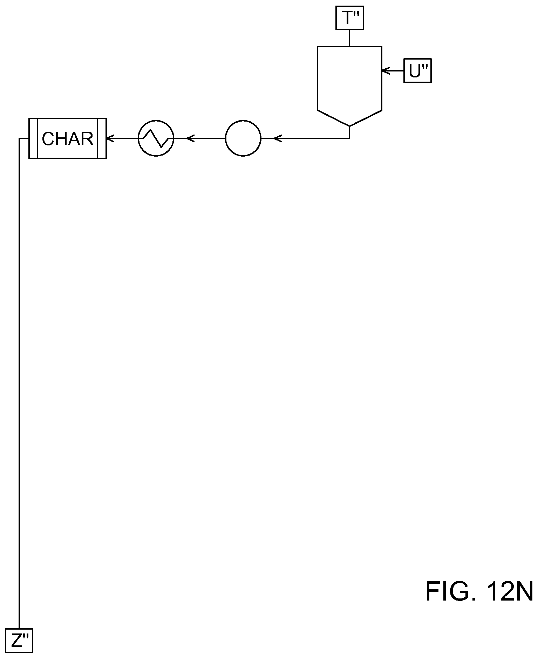

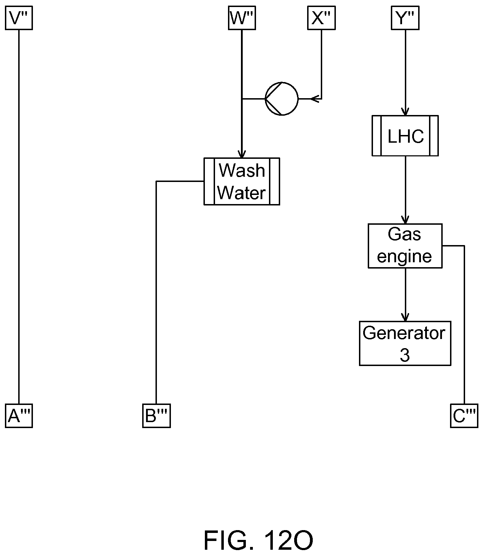

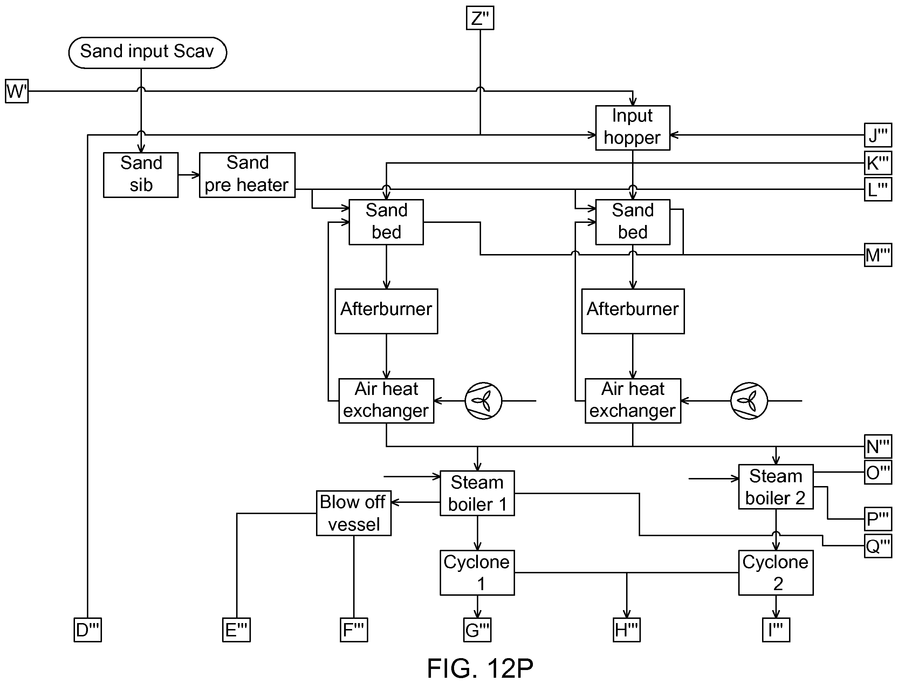

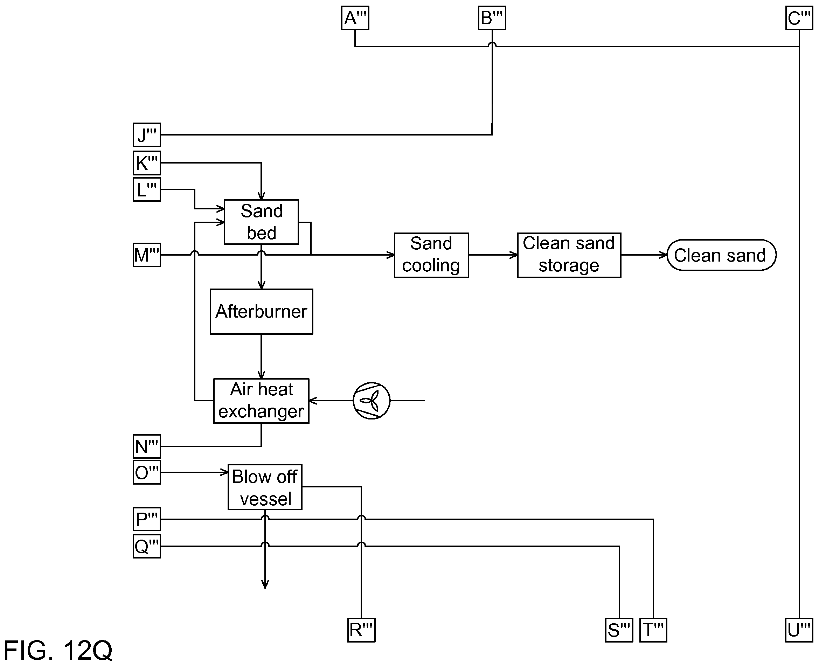

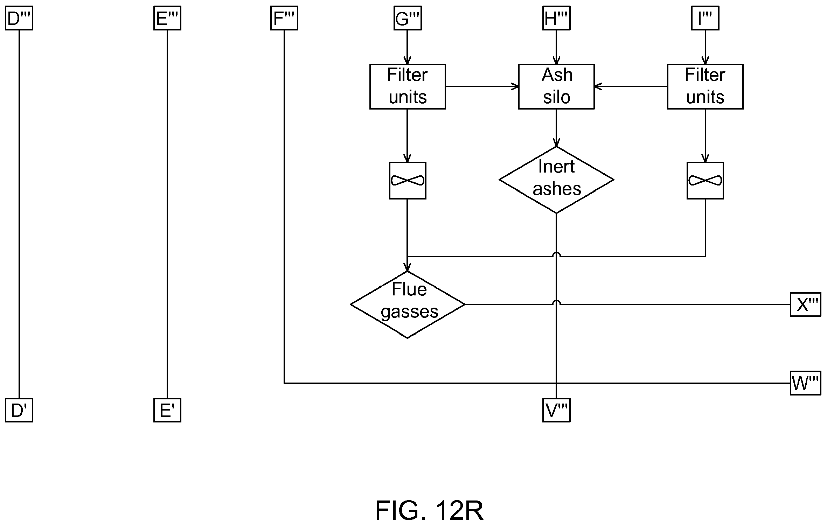

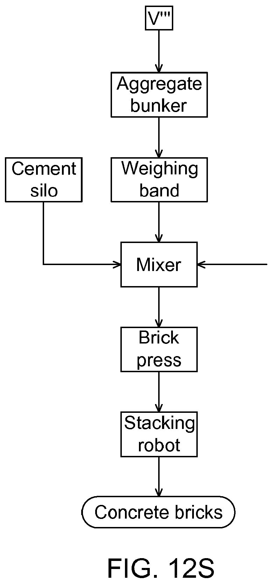

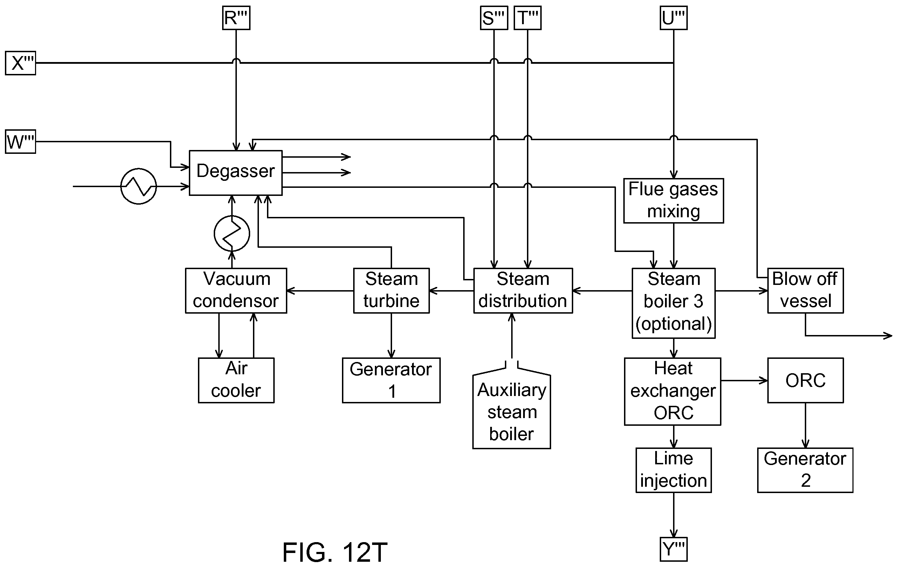

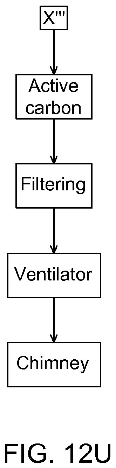

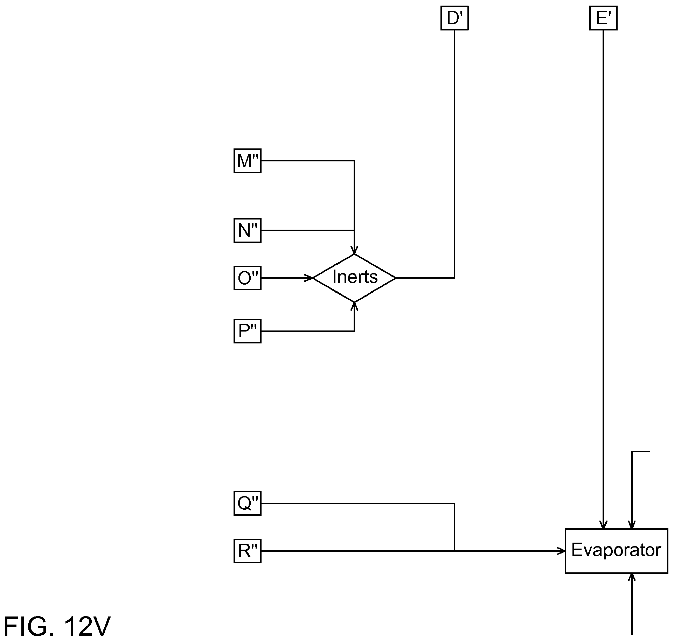

Referring now to Figs 12A to 12V, an example of an overall integrated process can be seen. These figures can be assembled together to form an overall process flow diagram, and each figure indicates where it should be generally positioned in relation to other figures for this assembly. In addition, it is noted that Figs 12A to 12V illustrate a detailed embodiment that can be modified in various ways. Further, one of more of the unit operations illustrated in these figures can be added to the more general processes or systems described and claimed herein. For example, Fig 12A shows that baled input MSW and loose input MSW can be fed as two separate streams, and thus the initial feed of MSW can be provided as two or more separate feed streams into the process. As another example, Fig 12A shows a weighing system, and this unit operation can be added into the more general processes or systems described and claimed herein. Another example is the trommel screen in Fig 12A, which can be used as part of the separating step for separating the MSW into certain fractions/streams. The particular number of units and their specific integration as illustrated in Figs 12A to 12V are examples, and each unit operation can be added to the more general processes or systems described and claimed herein without necessarily requiring addition of such specific number or integration. Another example is that while Fig 12I shows two feed screws, five heat exchangers, a mixer, and a recycle line, it should be understood that this general process line could include one or more screw, one or more heat exchangers, one or more mixers, and/or one or more recycle lines prior to the feed being sent into the downstream units. As a further example, while Figs 12A to 12V may illustrate various specific streams being fed to specific units, it should be understood that in some implementations only some of the streams may be fed to certain units (e.g., three input streams are fed to the hopper at the top of Fig 12P, but it should be noted that only one or two of such streams could be supplied to the hopper specifically and/or to the thermal reactor in general.

Referring to Fig 5, an example of a complete process including mass balance information is provided. Depending on the composition of the MSW and the particular unit operations that are implemented, the mass balance may be modified.

Figs 12A to 12V, viewed together, also illustrate an example overall process for treating municipal solid waste.

## Claims

1. A process for treating a solid waste containing material, comprising:
separating the solid waste containing material to produce at least a plastics fraction (18), an organic fraction (20), and a residual fraction (22);
subjecting at least a portion of the plastics fraction to plastics processing (32) to produce at least one hydrocarbon product;
subjecting a first portion (74) of the organics fraction to bio-ethanol production;
subjecting a second portion (76) of the organics fraction to bio-coal production; and
supplying at least a portion of the residual fraction to a thermal reactor (108) to produce an inert material (110) and heat energy (203),
wherein said bio-ethanol production comprises:
enzymatically treating (80) the first portion (74) of the organics fraction to produce a sugar-enriched stream (84);
fermenting (86) at least a portion of the sugar-enriched stream to produce an ethanol containing stream (88); and
separating (90) ethanol from the ethanol containing stream to produce a bio-ethanol product (92),
and wherein said bio-coal production comprises:
subjecting the second portion (76) of the organics fraction to hydrothermal carbonization (94) to produce a thermally treated stream such as coal sludge; and
removing water from the thermally treated stream to produce a bio-coal product (104).

2. The process of claim 1, wherein the solid waste containing material comprises municipal solid waste (MSW).

3. The process of claim 2 for treating municipal solid waste (MSW), comprising:
separating the MSW to produce a plastics fraction (18), an organics fraction (20), a directly recyclable materials fraction (16) and a residual fraction (22);
subjecting at least a portion of the plastics fraction to plastics processing (32) to produce at least one hydrocarbon product Z , such as a J Z diesel product, paraffin product and/or hydrocarbon product suitable for use as an end product or base product in the petrochemical industry;
subjecting a first portion (74) of the organics fraction to bio-ethanol production, comprising:
enzymatically treating (80) the first portion (74) of the organics fraction to produce a sugar-enriched stream (84);
fermenting (86) at least a portion of the sugar-enriched stream to produce an ethanol containing stream (88); and
separating (90) ethanol from the ethanol containing stream to produce a bio-ethanol product (92);
subjecting a second portion (76) of the organics fraction to bio-coal production, comprising:
subjecting the second portion (76) of the organics fraction to hydrothermal carbonization (94) to produce a thermally treated stream (e.g., coal sludge); and
removing water from the thermally treated stream to produce a bio-coal product (104);
separating the directly recyclable materials fraction (16) into a ferro-materials fraction (26) and a non ferro-materials fraction (28), and recycling the same; and
supplying at least a portion of the residuals fraction to a thermal reactor such as a fluid sand bed reactor, (108) to produce an inert material (110) and heat energy (203).

4. The process according to any one of claims 1 to 3, further comprising reusing recovered heat energy in the process.

5. A system for treating municipal solid waste (MSW), comprising:
a separation unit (14) for separating the MSW (12) to produce a plastics fraction (18), an organics fraction (20), a directly recyclable ferro-materials fraction (26), a directly recyclable non-ferro-materials (28) and a residual fraction (22), wherein the directly recyclable ferro-materials fraction (26) and the directly recyclable non-ferro-materials (28) are recycled;
a plastics-to-oil processing unit for converting at least a portion of the plastics fraction to into at least one hydrocarbon product;
a bioethanol production unit for receiving a first portion (74) of the organics fraction to produce bio-ethanol;
a bio-coal production unit for receiving a second portion (76) of the organics fraction to produce bio-coal, said bio-coal production unit comprising:
a hydrothermal carbonization unit (98) that receives the second portion (76) of the organics fraction to produce a thermally treated stream; and
a water removal unit (100) that receives the thermally treated stream to produce a bio-coal product (104) and a recovered water stream;
and
a thermal reactor Z such as a scavenger unit, (108) receiving the residuals fraction and producing an inert material and heat energy (203), and reusing the recovered heat energy (203) in the process.

6. The system according to claim 5, further comprising a plastics recycle unit (34) for subjecting a portion of the plastics fraction to a plastics recycling operation in which at least one plastic compound is separated from the plastics fraction, wherein the at least one plastic compound comprises at least three separate categories including PET, PVC and PE/PP.

7. The system according to claim 5 or 6, further comprising a heat energy recovery assembly (114) for recovering heat energy from the thermal reactor(108) and reusing the recovered heat energy in the process.

## Patentansprüche

1. Verfahren zur Behandlung eines festen Abfall enthaltenden Materials, umfassend:
Trennen des festen Abfall enthaltenden Materials, um wenigstens eine Kunststofffraktion (18), eine Fraktion von organischen Stoffen (20) und eine Reststofffraktion (22) zu erzeugen;
Unterwerfen wenigstens eines Teils der Kunststofffraktion an Kunststoffverarbeitung (32), um wenigstens ein Kohlenwasserstoffprodukt zu erzeugen;
Unterwerfen eines ersten Teils (74) der Fraktion von organischen Stoffen an Bioethanolproduktion;
Unterwerfen eines zweiten Teils (76) der Fraktion von organischen Stoffen an Biokohleproduktion; und
Zuführen wenigstens eines Teils der Reststofffraktion an einen thermischen Reaktor (108), um ein inertes Material (110) und Wärmeenergie (203) zu erzeugen;
wobei die Bioethanolproduktion umfasst:
enzymatische Behandlung (80) des ersten Teils (74) der Fraktion von organischen Stoffen, um einen zuckerangereicherten Strom (84) zu erzeugen;
Fermentieren (86) wenigstens eines Teils des zuckerangereicherten Stroms, um einen ethanolhaltigen Strom (88) zu erzeugen; und
Abtrennen (90) von Ethanol aus dem ethanolhaltigen Strom, um ein Bioethanolprodukt (92) zu erzeugen;
und wobei die Biokohleproduktion umfasst:
Unterwerfen des zweiten Teils (76) der Fraktion von organischen Stoffen an hydrothermische Carbonisierung (94), um einen wärmebehandelten Strom, wie z. B. Kohleschlamm, zu erzeugen; und
Entfernen von Wasser aus dem wärmebehandelten Strom, um ein Biokohleprodukt (104) zu erzeugen.

2. Verfahren gemäß Anspruch 1, wobei das festen Abfall enthaltende Material festen kommunalen Abfall ("municipal solid waste", MSW) umfasst.

3. Verfahren gemäß Anspruch 2 zur Behandlung von festem kommunalem Abfall (MSW), umfassend:
Trennen des MSW, um eine Kunststofffraktion (18), eine Fraktion von organischen Stoffen (20), eine Fraktion von direkt recyclingfähigen Materialien (16) und eine Reststofffraktion (22) zu erzeugen;
Unterwerfen wenigstens eines Teils der Kunststofffraktion an Kunststoffverarbeitung (32), um wenigstens ein Kohlenwasserstoffprodukt, wie z. B. ein Dieselprodukt, Paraffinprodukt und/oder Kohlenwasserstoffprodukt, das für die Verwendung als Endprodukt oder Grundprodukt in der petrochemischen Industrie geeignet ist, zu erzeugen;
Unterwerfen eines ersten Teils (74) der Fraktion von organischen Stoffen an Bioethanolproduktion, umfassend:
enzymatische Behandlung (80) des ersten Teils (74) der Fraktion von organischen Stoffen, um einen zuckerangereicherten Strom (84) zu erzeugen;
Fermentieren (86) wenigstens eines Teils des zuckerangereicherten Stroms, um einen ethanolhaltigen Strom (88) zu erzeugen; und
Abtrennen (90) von Ethanol aus dem ethanolhaltigen Strom, um ein Bioethanolprodukt (92) zu erzeugen;
Unterwerfen eines zweiten Teils (76) der Fraktion von organischen Stoffen an Biokohleproduktion, umfassend:
Unterwerfen des zweiten Teils (76) der Fraktion von organischen Stoffen an hydrothermische Carbonisierung (94), um einen wärmebehandelten Strom (z. B. Kohleschlamm) zu erzeugen; und
Entfernen von Wasser aus dem wärmebehandelten Strom, um ein Biokohleprodukt (104) zu erzeugen;
Trennen der Fraktion von direkt recyclingfähigen Materialien (16) in eine Fraktion von Ferromaterialien (26) und eine Fraktion von Nichtferromaterialien (28) und Recyceln davon; und
Zuführen wenigstens eines Teils der Reststofffraktion an einen thermischen Reaktor, wie z. B. einen Sand-Fließbettreaktor (108), um ein inertes Material (110) und Wärmeenergie (203) zu erzeugen.

4. Verfahren gemäß einem der Ansprüche 1 bis 3, ferner umfassend Wiederverwendung von gewonnener Wärmeenergie bei dem Verfahren.

5. System zur Behandlung von festem kommunalem Abfall (MSW), umfassend:
eine Trenneinheit (14) zum Trennen des MSW (12), um eine Kunststofffraktion (18), eine Fraktion von organischen Stoffen (20), eine Fraktion von direkt recyclingfähigen Ferromaterialien (26), direkt recyclingfähige Nichtferromaterialien (28) und eine Reststofffraktion (22) zu erzeugen, wobei die Fraktion von direkt recyclingfähigen Ferromaterialien (26) und die direkt recyclingfähigen Nichtferromaterialien (28) recycelt werden;
eine Kunststoff-zu-ÖI-Verarbeitungseinheit zum Umwandeln wenigstens eines Teils der Kunststofffraktion in wenigstens ein Kohlenwasserstoffprodukt,
eine Bioethanolproduktionseinheit zum Aufnehmen eines ersten Teils (74) der Fraktion von organischen Stoffen, um Bioethanol zu erzeugen;
eine Biokohleproduktionseinheit zum Aufnehmen eines zweiten Teils (76) der Fraktion von organischen Stoffen, um Biokohle zu erzeugen, wobei die Biokohleproduktionseinheit umfasst:
eine Einheit zur hydrothermischen Carbonisierung (98), die den zweiten Teil (76) der Fraktion von organischen Stoffen aufnimmt, um einen wärmebehandelten Strom zu erzeugen; und
eine Wasserentfernungseinheit (100), die den wärmebehandelten Strom aufnimmt, um ein Biokohleprodukt (104) und einen Strom von rückgewonnenem Wasser zu erzeugen;
und
einen thermischen Reaktor (108), wie z. B. eine Scavenger-Einheit, die die Reststofffraktion aufnimmt und ein inertes Material und Wärmeenergie (203) erzeugt und die gewonnene Wärmeenergie (203) bei dem Verfahren wiederverwendet.

6. System gemäß Anspruch 5, ferner umfassend eine Kunststoffrecyclingeinheit (34) zum Unterwerfen eines Teils der Kunststofffraktion an einen Kunststoffrecyclingvorgang, bei dem wenigstens eine Kunststoffverbindung von der Kunststofffraktion abgetrennt wird, wobei die wenigstens eine Kunststoffverbindung wenigstens drei getrennte Kategorien, einschließlich PET, PVC und PE/PP, umfasst.

7. System gemäß Anspruch 5 oder 6, ferner umfassend eine Wärmeenergiegewinnungsanlage (114) zur Gewinnung von Wärmeenergie aus dem thermischen Reaktor (108) und Wiederverwenden der gewonnenen Wärmeenergie bei dem Verfahren.

## Revendications

1. Procédé destiné à traiter un matériau contenant des déchets solides, comprenant:
la séparation du matériau contenant des déchets solides pour produire au moins une fraction de matières plastiques (18), une fraction organique (20) et une fraction résiduelle (22) ;
soumettre au moins une partie de la fraction de matières plastiques à une transformation (32) de matières plastiques pour produire au moins un produit de type hydrocarbure ;
soumettre une première partie (74) de la fraction de composés organiques à une production de bioéthanol ;
soumettre une seconde partie (76) de la fraction de composés organiques à une production de biocharbon ; et
l'envoi d'au moins une partie de la fraction résiduelle vers un réacteur thermique (108) pour produire un matériau inerte (110) et de l'énergie thermique (203), dans lequel ladite production de bioéthanol comprend :
le traitement enzymatique (80) de la première partie (74) de la fraction de composés organiques pour produire un flux enrichi en sucres (84) ;
la fermentation (86) d'au moins une partie du flux enrichi en sucres pour produire un flux contenant de l'éthanol (88) ; et
la séparation (90) d'éthanol à partir du flux contenant de l'éthanol pour produire un produit de type bioéthanol (92),
et dans lequel ladite production de biocharbon comprend :
soumettre la seconde partie (76) de la fraction de composés organiques à une carbonisation hydrothermale (94) pour produire un flux traité thermiquement tel que de la boue de charbon ; et
l'élimination d'eau du flux traité thermiquement pour produire un produit de type biocharbon (104).

2. Procédé selon la revendication 1, dans lequel le matériau contenant des déchets solides comprend des déchets solides municipaux (DSM).

3. Procédé selon la revendication 2 destiné à traiter des déchets solides municipaux (DSM), comprenant :
la séparation des DSM pour produire une fraction de matières plastiques (18), une fraction de composés organiques (20), une fraction de matériaux directement recyclables (16) et une fraction résiduelle (22) ;
soumettre au moins une partie de la fraction de matières plastiques à une transformation (32) de matières plastiques pour produire au moins un produit de type hydrocarbure, tel qu'un produit de type diesel, un produit de type paraffine et/ou un produit de type hydrocarbure approprié pour être utilisé en tant que produit final ou produit de base dans l'industrie pétrochimique ;
soumettre une première partie (74) de la fraction de composés organiques à une production de bioéthanol, comprenant :
le traitement enzymatique (80) de la première partie (74) de la fraction de composés organiques pour produire un flux enrichi en sucres (84) ;
la fermentation (86) d'au moins une partie du flux enrichi en sucres pour produire un flux contenant de l'éthanol (88) ; et
la séparation (90) d'éthanol à partir du flux contenant de l'éthanol pour produire un produit de type bioéthanol (92) ;
soumettre une seconde partie (76) de la fraction de composés organiques à une production de biocharbon, comprenant :
soumettre la seconde partie (76) de la fraction de composés organiques à une carbonisation hydrothermale (94) pour produire un flux traité thermiquement (par exemple de la boue de charbon) ; et
l'élimination d'eau du flux traité thermiquement pour produire un produit de type biocharbon (104) ;
la séparation de la fraction de matériaux directement recyclables (16) en une fraction de matériaux ferreux (26) et une fraction de matériaux non ferreux (28) et le recyclage de celles-ci ; et
l'envoi d'au moins une partie de la fraction de composés résiduels vers un réacteur thermique, tel qu'un réacteur à lit de sable fluidisé (108), pour produire un matériau inerte (110) et de l'énergie thermique (203).

4. Procédé selon l'une quelconque des revendications 1 à 3, comprenant en outre la réutilisation dans le procédé de l'énergie thermique récupérée.

5. Système destiné à traiter des déchets solides municipaux (DSM), comprenant :
une unité de séparation (14) destinée à séparer les DSM (12) pour produire une fraction de matières plastiques (18), une fraction de composés organiques (20), une fraction de matériaux ferreux directement recyclables (26), des matériaux non ferreux directement recyclables (28) et une fraction résiduelle (22), la fraction de matériaux ferreux directement recyclables (26) et les matériaux non ferreux directement recyclables (28) étant recyclés ;
une unité de transformation de matières plastiques en huile destinée à convertir au moins une partie de la fraction de matières plastiques en au moins un produit de type hydrocarbure ;
une unité de production de bioéthanol destinée à recevoir une première partie (74) de la fraction de composés organiques pour produire du bioéthanol ;
une unité de production de biocharbon destinée à recevoir une seconde partie (76) de la fraction de composés organiques pour produire du biocharbon, ladite unité de production de biocharbon comprenant :
une unité de carbonisation hydrothermale (98) qui reçoit la seconde partie (76) de la fraction de composés organiques pour produire un flux traité thermiquement ; et
une unité d'élimination d'eau (100) qui reçoit le flux traité thermiquement pour produire un produit de type biocharbon (104) et un flux d'eau récupérée ; et
un réacteur thermique, tel qu'une unité épuiseuse (108), recevant la fraction de composés résiduels et produisant un matériau inerte et de l'énergie thermique (203) et réutilisant dans le procédé l'énergie thermique récupérée (203).

6. Système selon la revendication 5, comprenant en outre une unité de recyclage (34) de matières plastiques destinée à soumettre une partie de la fraction de matières plastiques à une opération de recyclage de matières plastiques dans laquelle au moins un composé plastique est séparé de la fraction de matières plastiques, dans lequel l'au moins un composé plastique comprend au moins trois catégories séparées dont le PET, le PVC et le PE/PP.

7. Système selon la revendication 5 ou 6, comprenant en outre un ensemble de récupération d'énergie thermique (114) destiné à récupérer de l'énergie thermique à partir du réacteur thermique (108) et réutiliser dans le procédé l'énergie thermique récupérée.
